# EUROPEAN PATENT SPECIFICATION

(11) **EP 3 234 084 B1**
(45) Date of publication and mention of the grant of the patent: **25.10.2023**
(21) Application number: 15871253.9
(22) Date of filing: 18.12.2015
(51) Int. Cl.: C07C 67/03, C07C 67/08, C07C 69/52, C11C 3/10, C10L 11/00, C10L 1/02, C11C 3/00

(54) **SYSTEMS AND METHODS FOR THE NON-CATALYTIC PRODUCTION OF BIODIESEL FROM OILS**
SYSTEME UND VERFAHREN ZUR NICHTKATALYTISCHEN HERSTELLUNG VON BIODIESEL AUS ÖLEN
SYSTÈMES ET PROCÉDÉS DE PRODUCTION NON CATALYTIQUE DE BIODIESEL À PARTIR D'HUILES

(30) Priority: 19.12.2014 US 201462094333 P; 12.05.2015 US 201562160156 P; 01.09.2015 US 201562212855 P
(43) Date of publication of application: 25.10.2017
(73) Proprietor: INVENTURE INTERNATIONAL (PTE) LIMITED, Singapore 308899 (SG)
(72) Inventor: SUTTERLIN, William Rusty, Tuscaloosa, Alabama 35401 (US); LONG, Ryan, Tuscaloosa, Alabama 35405 (US); GRAY III, Lester Trummer, Cottondale, Alabama 35453 (US); SAWYER, Hayden, Tuscaloosa, Alabama 35401 (US)
(74) Representative: Cantaluppi, Stefano
(86) International application number: PCT/US2015/066932
(87) International publication number: WO 2016/100944

(56) References cited:
- EP-A1- 0 985 654
- WO-A1-2007/058485
- US-A1- 2007 277 432
- US-A1- 2008 312 468
- US-A1- 2011 271 585
- S SAKA ET AL: "Non-catalytic biodesel fuel production with supercritical methanol technologies", JOURNAL OF SCIENTIFIC & INDUSTRIAL RESEARCH, vol. 65, 1 May 2006 (2006-05-01), pages 420-425, XP055457074,
- SAKA ET AL.: 'Non-catalytic biodesel fuel production with supercritical methanol technologies' JOURNAL OF SCIENTIFIC & INDUSTRIAL RESEARCH, [Online] vol. 65, 01 May 2006, pages 420 - 425, XP055457074 Retrieved from the Internet: <URL:http://nopr.niscair.res.in/bitstream/1 23456789/4842/1/JSIR% 2065(5)%20420-425.pdf> [retrieved on 2016-02-12]

## Description

### TECHNICAL FIELD

This invention generally relates to the economically efficient preparation of a biodiesel or a fat-based diesel fuel from a feedstock comprising a palm oil fatty acid distillate (PFAD) or a feedstock comprising a palm oil, dende oil, an oil from a plant of the genus Elaeis or Attalea.

### BACKGROUND

Biodiesel is a renewable fuel that can be blended with, or replace, conventional diesel fuel for combustion in diesel engines. Current commercial methods for the production of biodiesel involve subjecting natural oils (e.g. soybean oil or palm oil) to a transesterification process in which triglycerides within the natural oil feedstock are reacted with alcohol in the presence of a basic catalyst, e.g. sodium hydroxide, or a two-stage process wherein the oil is subjected to a first acid-catalyzed esterification reaction and then a second base-catalyzed transesterification reaction. The composition of the biodiesel product depends on the alcohol used in the transesterification reaction. For example, if methanol is the selected alcohol, the resultant biodiesel will be comprised of fatty acid methyl esters (FAME). If ethanol is the selected alcohol, the resultant biodiesel product will be comprised of fatty acid ethyl esters (FAEE).

In addition to biodiesel, the resulting product from the catalyzed transesterification of natural oils also contains glycerol (i.e. glycerin) and unreacted alcohol. The glycerol product is typically contaminated and unsuitable for use as high-value "food-grade" glycerol. In order to obtain fuel-grade biodiesel product, e.g. a biodiesel meeting the specifications set forth in the American Society of Testing and Manufacturing (ASTM) Specification D6751, the transesterification product mixture must undergo further processing in order to separate the fatty acid alkyl esters from the reaction by-products such as glycerol, unreacted alcohol, water, free fatty acids, salts, and light and heavy organics. Conventional separation techniques, most typically liquid-liquid-type batch separation techniques, are time consuming, maintenance intensive, and economically inefficient.

Conventional biodiesel production techniques are also limited in their ability to process oils with high free fatty acid content, e.g. oils with free fatty acid contents of 10% or greater. This is primarily due to the tendency of free fatty acids to react with the catalyst, increasing catalyst replacement costs and lowering overall biodiesel yields. Such shortcomings limit the growth of the biodiesel market.

Saka, S., Kusdiana, D., and Minami, E., "Non-catalytic Biodiesel Fuel Production with Supercritical Methanol Technologies" J. Sci. Ind. Res. 65 (2006) 420-425 discloses a method for producing biodiesel using supercritical methanol without any catalyst.

WO2007058485 discloses a method for producing biodiesel using supercritical alcohols for esterifying oils and fats.

EP0985654 discloses a method for preparing fatty acid esters useful as fuels by reacting fats and oils with an alcohol in the absence of a catalyst.

### SUMMARY

According to the invention it is provided a method or an industrial process for producing a biodiesel or a fat-based diesel fuel according to claim 1. Further advantageous features of the method are set forth in the dependent claims.

According to the invention it is provided a method or an industrial processes for producing a biodiesel or a fat-based diesel fuel from a feedstock comprising a palm oil fatty acid distillate (PFAD) or a feedstock comprising a palm oil, dende oil, an oil from a plant of the genus *Elaeis* or *Attalea,* the method comprising:
(a) providing a palm oil fatty acid distillate (PFAD) or a feedstock comprising a palm oil, dende oil, an oil from a plant of the genus *Elaeis* or *Attalea;*
(b) providing an alcohol, optionally a methanol;
(c) subjecting the PFAD or feedstock of (a) to an esterification/ transesterification reaction with the alcohol under conditions comprising at or above the critical temperature and pressure of the alcohol in the absence of any catalyst, wherein free fatty acids (FFAs) in the PFAD or feedstock undergo an esterification reaction with the alcohol to generate a product comprising fatty acid alkyl esters, and the glycerides undergo a transesterification reaction with the alcohol to generate a product comprising fatty acid alkyl esters; and
(d) separating the product generated in the esterification/transesterification reaction of step (c) into a "light" fraction comprising the lighter alkyl esters, optionally alkyl esters with 16 or fewer carbons, and a "heavy" fraction comprising heavy alkyl esters, optionally alkyl esters with more than 16 carbons, and any unreacted FFAs,

wherein optionally the esterification/transesterification reaction product is distilled, optionally in a conventional distillation column or equivalent, to separate the lighter fatty acid alkyl esters from the other components of the reaction product,
and optionally if PFAD is the feedstock, the majority of the fatty acid alkyl esters comprise alkyl esters of palmitic acid, optionally methyl palmitate if methanol is the alcohol used in the reaction, and the majority of the fatty acids present in the feedstock with 16 or fewer carbons comprise palmitic acid,
and optionally the "bottoms" in the distillation column comprise heavy fatty acid alkyl esters (alkyl esters with more than 16 carbons), unreacted FFAs, any unreacted esters e.g. mono- di- and triglycerides, phospholipids, and any other unsaponifiable material in the feedstock, optionally sterols, vitamin E compounds (tocopherols and/or tocotrienols), squalene, or other compounds.

In alternative embodiments, the methods and industrial processes further comprise subjecting the "heavy" fraction comprising heavy alkyl esters, or the bottoms of the distillation column, to a second esterification/transesterification reaction with a supercritical alcohol, or at or above the critical temperature and pressure of the alcohol in the absence of any catalyst,
wherein approximately 95% of the unreacted FFAs and esters from the first esterification/transesterification reaction are converted to fatty acid alkyl esters,
and optionally the product mixture generated in the second esterification/ transesterification reaction generates a product mixture comprising less than about 1% FFA.

In alternative embodiments, the methods and industrial processes further comprise processing the second product mixture to separate the fatty acid alkyl esters from the remaining components of the product mixture using, optionally distilling or separating to generate an alkyl ester product that is suitable for use as an ASTM B 100 biodiesel.

In alternative embodiments, the alkyl ester biodiesel product separated in the second distillation or other separation technique are mixed or combined with the alkyl esters separated from the first reaction product to increase the overall biodiesel yield of the process.

In alternative embodiments, the methods and industrial processes further comprise subjecting the "heavy" fraction comprising heavy alkyl esters, or the bottoms of the distillation column, to an acid-catalyzed alcohol esterification reaction (instead of a second esterification/ transesterification reaction with a supercritical alcohol) comprising a strong acid cation exchange resin, wherein optionally the reaction is an alcohol esterification reaction in the presence of a strong acid cation resin or equivalent, the resin acting as an acid catalyst of the reaction.

In alternative embodiments, the methods and industrial processes further comprise:
(a) mixing the bottoms (comprising the unreacted FFAs, any unreacted esters, optionally mono- di- and triglycerides, phospholipids, and any other unsaponifiable material in the feedstock, optionally sterols, vitamin E compounds such as tocopherols and/or tocotrienols, squalene, or other compounds, with an alcohol, optionally methanol, to form an alcohol/bottoms mixture; and
(b) heating the alcohol/bottoms mixture, optionally using a heat exchanger, optionally, a heat exchanger operationally connected to another portion of the process, to between about 80 and 100 °C.

In alternative embodiments, the methods and industrial processes further comprise passing or pumping the alcohol/bottoms mixture through a pipe or other suitable container or vessel comprising a cation resin (optionally a packed cation resin) or equivalent until substantially all of the saponifiable material in the mixture is converted to fatty acid alkyl esters.

In alternative embodiments, the methods and industrial processes further comprise flashing off unreacted alcohol, and optionally recovering and recycling the alcohol.

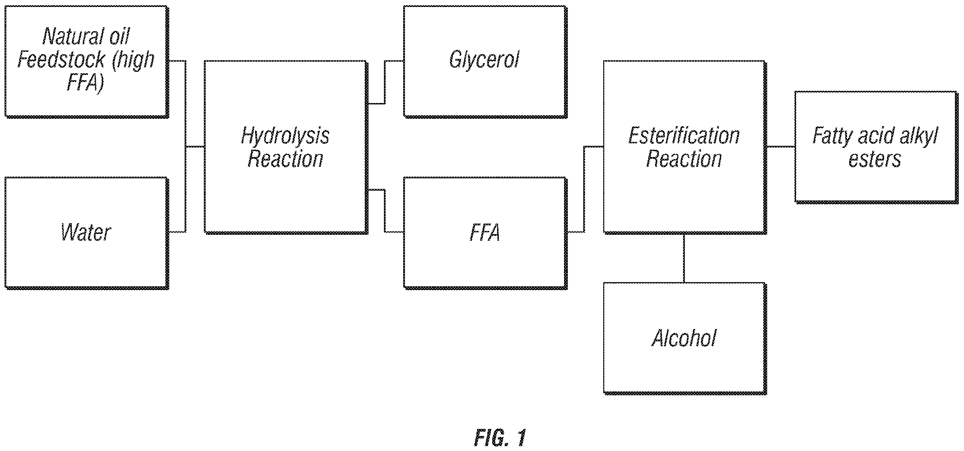

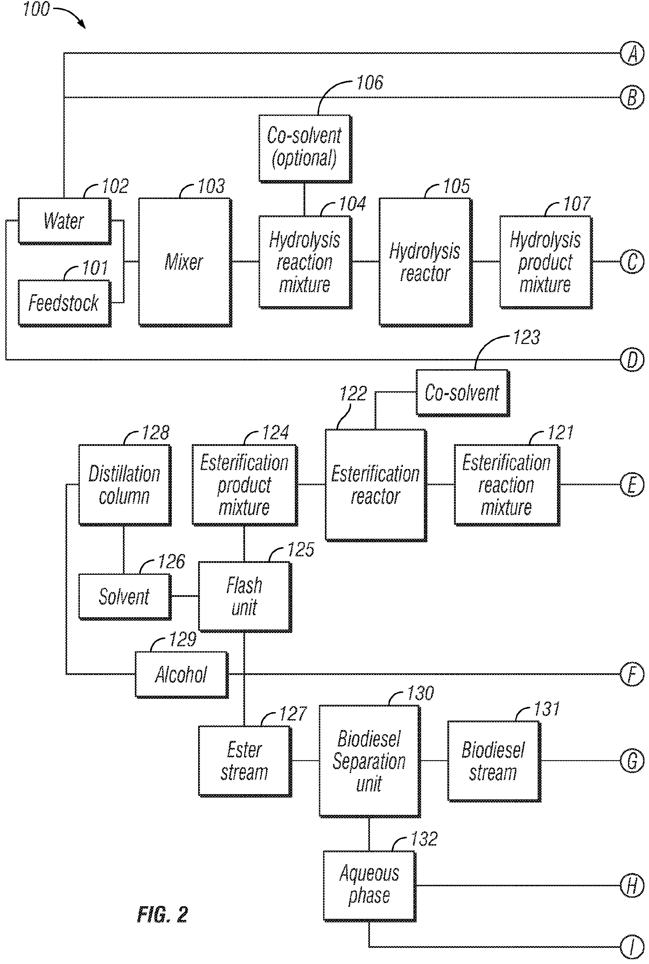

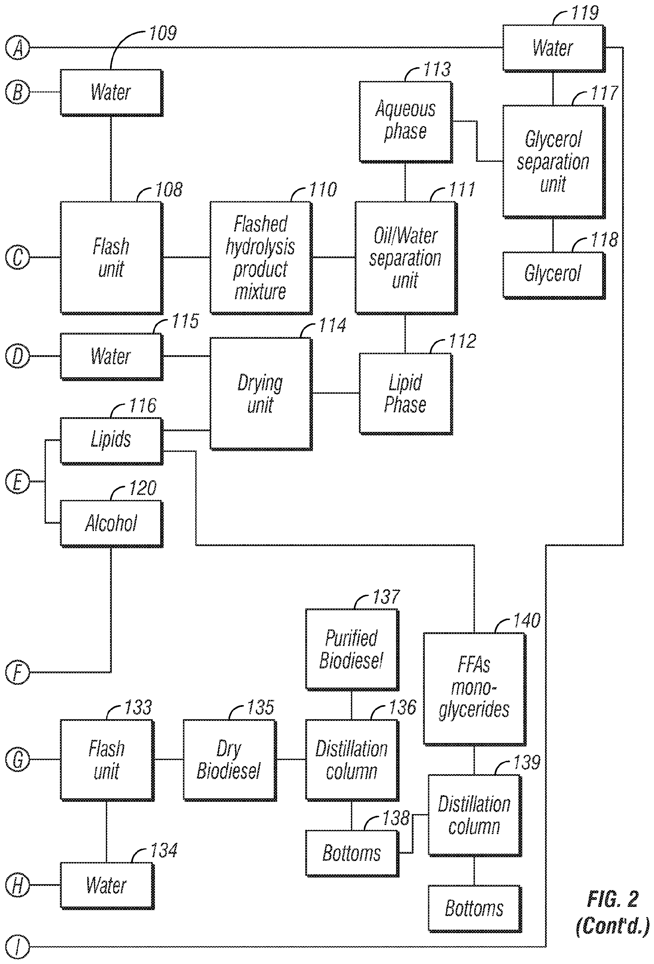

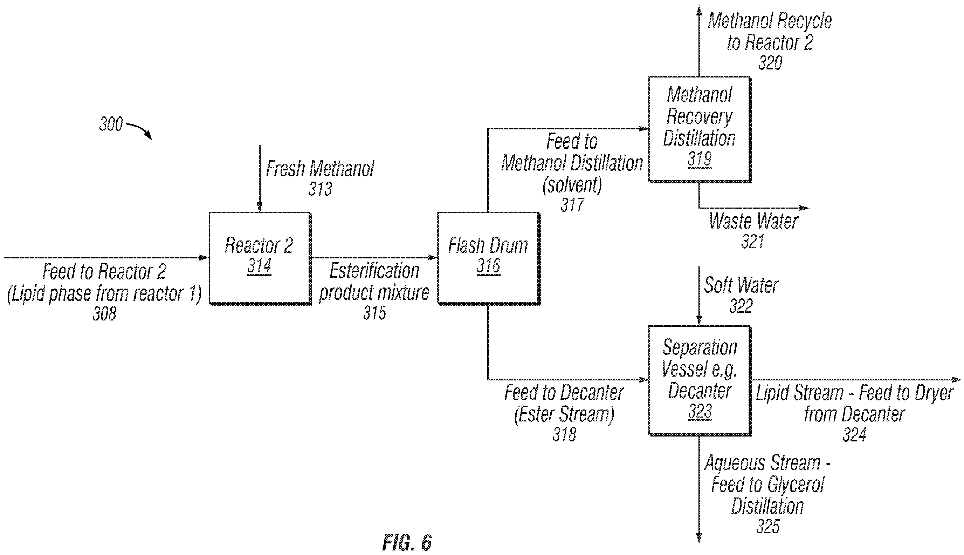

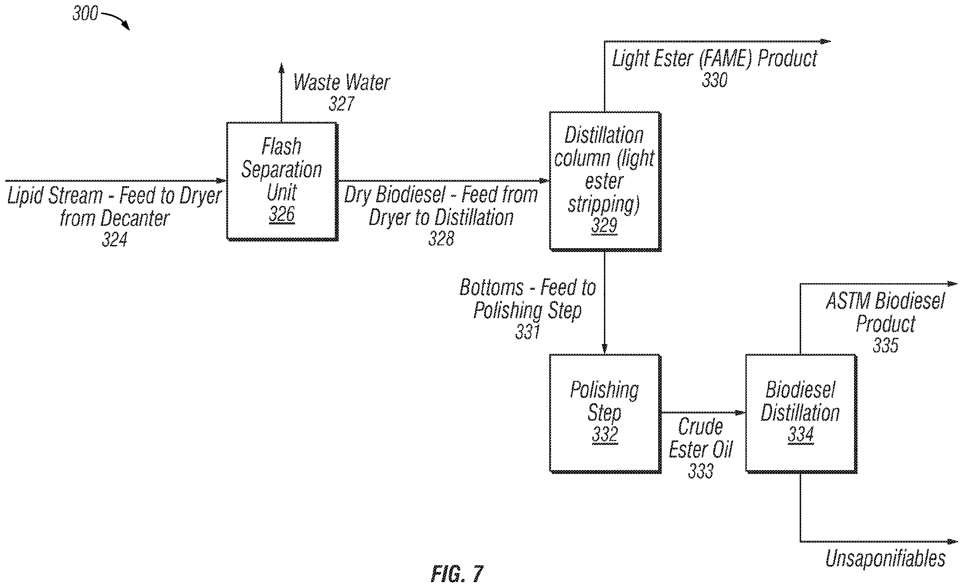

In alternative embodiments, provided are methods and industrial processes comprising a process as described in any of all or part of Figure 1, Figure 2, Figure 3, Figure 4, Figure 5, Figure 6 and/or Figure 7.

Are here described also systems, bioreactors and equivalent products of manufacture configured to operate or manufactured for carrying out a method or industrial process as provided herein.

The details of one or more exemplary embodiments are set forth in the description below. Other features, objects, and advantages of the invention will be apparent from the description and the claims.

### BRIEF DESCRIPTION OF THE DRAWINGS

The drawings set forth herein are illustrative of embodiments as provided herein and are not meant to limit the scope of the invention as encompassed by the claims.
Fig. 1 is a simplified schematic diagram of exemplary methods, not part of the invention, as described herein comprising generating biodiesel and/or other products e.g. fatty acid alkyl esters, free fatty acids, glycerol or combinations thereof from natural oil feedstocks.
Fig. 2 is a schematic diagram of exemplary methods, not part of the invention, as described herein comprising generating biodiesel and/or other products e.g. fatty acid alkyl esters, free fatty acids, glycerol or combinations thereof from natural oil feedstocks.
Fig. 3 is a schematic diagram of exemplary methods as provided herein comprising generating biodiesel and/or other products e.g. fatty acid alkyl esters, free fatty acids, glycerol or combinations thereof, from natural oil feedstocks.
Fig. 4 is a continuation of the schematic diagram of Fig. 3 of exemplary methods as provided herein comprising generating biodiesel and/or other products e.g. fatty acid alkyl esters, free fatty acids, glycerol or combinations thereof, from natural oil feedstocks.
Fig. 5 is a schematic diagram of exemplary methods as provided herein comprising generating biodiesel and/or other products e.g. fatty acid alkyl esters, free fatty acids, glycerol or combinations thereof, from natural oil feedstocks.
Fig. 6 is a continuation of the schematic diagram of Fig. 5 of exemplary methods as provided herein comprising generating biodiesel and/or other products e.g. fatty acid alkyl esters, free fatty acids, glycerol or combinations thereof, from natural oil feedstocks.
Fig. 7 is a continuation of the schematic diagram of Fig. 6 of exemplary methods as provided herein comprising generating biodiesel and/or other products e.g. fatty acid alkyl esters, free fatty acids, glycerol or combinations thereof, from natural oil feedstocks.

Reference will now be made in detail to various exemplary embodiments of the invention. The following detailed description is provided to give the reader a better understanding of certain details of aspects and embodiments of the invention, and should not be interpreted as a limitation on the scope of the invention.

Like reference symbols in the various drawings indicate like elements.

Reference will now be made in detail to various exemplary embodiments, examples of which are illustrated in the accompanying drawings. The following detailed description is provided to give the reader a better understanding of certain details of aspects and embodiments as provided herein, and should not be interpreted as a limitation on the scope of the invention.

### DETAILED DESCRIPTION

According to the invention are provided processes for the economically efficient preparation of high-quality biodiesel has defined in claim 1. without the use of any catalyst, the use of which is the standard in the art for producing biodiesel products from natural oils. In alternative embodiments, the systems and processes as provided herein are more economical and efficient than currently used approaches for the generation of biodiesel from natural oils.

It is described but does not form part of the invention a system for converting natural oil feedstocks into high-quality biodiesel without the use of any catalysts based on the composition of the natural oil feedstock used in the system. In alternative embodiments, the system is comprised of multiple operational units that are configured in alternative arrangements to accommodate the composition of the feedstock that is being converted to biodiesel and optionally other products.

In alternative embodiments, not within the scope of the invention, the system is configured in a single-stage process wherein a natural oil feedstock is mixed with an alcohol and the resulting mixture is heated and pressurized to above the critical temperature and pressure of the alcohol. The reaction conditions allow for the glycerides in the feedstock to undergo a transesterification reaction with the alcohol to generate fatty acid alkyl esters and the free fatty acids (FFAs) in the feedstock to undergo an esterification with the alcohol to generate fatty acid alkyl esters. The foregoing process is particularly suited to natural oil feedstocks comprising primarily glycerides but also a relatively high percentage (i.e. >10%) of FFAs. In conventional biodiesel processing approaches, the high FFA content of the feedstock decreases the efficiency of the process by reacting with the base (e.g. NaOH) catalyst.

In alternative embodiments, , not within the scope of the invention, the system is configured in a 2-stage process wherein the natural feedstock is mixed with water and the resulting mixture is heated and pressurized to allow for the glycerides in the feedstock to react with the water, thereby hydrolyzing the glycerides to glycerol and FFAs. The resulting FFAs are then mixed with alcohol and the resulting mixture is heated and pressurized to above the critical temperature and pressure of the alcohol. The reaction conditions allow for the FFAs in the feedstock to undergo an esterification with the alcohol to generate fatty acid alkyl esters.

In alternative embodiments, not within the scope of the invention, the system is configured to handle natural oil feedstocks comprising very high FFA content (e.g. >65% FFA), wherein a large percentage (e.g. >30% or about 40-50% or more) of the fatty acids in the feedstock are fully saturated, i.e. are without any double bonded carbons. In the foregoing arrangement, the system is configured such that the feedstock is mixed with an alcohol and the resulting mixture is mixture is heated and pressurized to above the critical temperature and pressure of the alcohol. The reaction conditions allow for the glycerides in the feedstock to undergo a transesterification reaction with the alcohol to generate fatty acid alkyl esters and the FFAs in the feedstock to undergo an esterification with the alcohol to generate fatty acid alkyl esters. In order to generate a sufficiently high-quality biodiesel product, the resulting product mixture is the subjected to a distillation step to separate any unreacted, saturated FFAs, e.g. methyl palmitate. The product wherein the unreacted saturated FFAs have been then been further processed to generated a high-quality biodiesel product and the separated, saturated FFAs can optionally be subjected to a second esterification reaction as described above and the resulting fatty acid alkyl esters can be mixed with the biodiesel product to increase the overall yields and efficiency of the system.

In alternative embodiments, not within the scope of the invention, the system can be configured to process natural oil feedstocks comprising primarily glycerides, wherein a large percentage (e.g. >30%) of the glycerides are saturated, e.g. crude palm oil. In this configuration, the system is configured in a 2-stage process such that the feedstock is subjected to a hydrolysis reaction in the first stage of the process to generate glycerol and FFAs, and the resulting FFAs are subjected to an esterification reaction in the second stage of the process to generate fatty acid alkyl esters. In order to generate a sufficiently high-quality biodiesel product, the esterification reaction product comprising the fatty acid alkyl esters is subjected to a distillation step wherein any unreacted, saturated FFAs, e.g. methyl palmitate. The product wherein the unreacted saturated FFAs have been then been further processed to generated a high-quality biodiesel product and the separated, saturated FFAs can optionally be subjected to a second esterification reaction as described above and the resulting fatty acid alkyl esters can be mixed with the biodiesel product to increase the overall yields and efficiency of the system.

In alternative embodiments, not within the scope of the invention, the system is "feedstock flexible" meaning that is can be configured to process natural oils with any free fatty acid content (i.e. any ratio of esters, e.g. glycerides, to free fatty acid) to and any fatty acid profile (e.g. percent saturated and unsaturated fatty acids). This represents a significant improvement over prior approaches to converting natural oil feedstocks to biodiesel in which systems are configured to handle a narrow range of feedstocks and are limited in their ability to process feedstocks comprising high FFA content, e.g. feedstocks with >10% FFA.

### Exemplary Single-Stage Processes

Provided are processes for the economically efficient preparation of high-quality biodiesel from feedstock comprising a palm oil fatty acid distillate (PFAD) or a feedstock comprising, wherein optionally the natural oil feedstock comprises a palm oil, dende oil, an oil from a plant of the genus Elaeis or Attalea. In alternative embodiments, provided are processes for the production of biodiesel meeting or exceeding the specifications for B100 biodiesel set forth in ASTM Specification D6751-14,. The feedstocks are subjected to a transesterification reaction with an alcohol under conditions at or above the critical temperature and pressure of the alcohol in the absence of any catalyst. The processes as provided herein are more economical and efficient than currently used approaches for the generation of biodiesel from natural oils.

In alternative embodiments, a "feedstock" is the starting material of a process or method as provided herein; and in alternative embodiments, noting that processes and methods as described herein are not limited by any particular mechanism of action, a "feedstock" is a starting material that undergoes a transesterification reaction to form a product mixture. The feedstock is from (e.g., isolated from) or derived from a palm oil, dende oil, an oil from a plant of the genus *Elaeis* or *Attalea* or any combination thereof.

In alternative embodiments, a "supercritical alcohol" is an alcohol at or above the critical temperature and pressure of the alcohol. At or above the critical point of the alcohol, distinct liquid and gas phases do not exist, and the phase-boundary between liquid and gas is terminated. Different alcohols have distinct critical temperatures and pressures. For example, methanol is supercritical at or above a temperature of approximately 240°C and pressure of approximately 81 bar (1173 psigg), or equivalents. Ethanol is supercritical at or above a temperature of approximately 241°C and pressure of approximately 61 bar (890psig), or equivalents.

In alternative embodiments, the alcohol used in a reaction or a process as provided herein contains between 1 and 5 carbons, or 1, 2, 3, 4, 5 or 6 or more carbons, e.g. methanol, ethanol, propanol, butanol, isobutanol, isopropyl alcohol or a combination thereof. In various other embodiments, conditions may be such that a higher alcohol containing more than 5 carbons are used. For purposes of this discussion, methanol is used as the alcohol, however those skilled in the art would understand that other alcohols could be used.

In alternative embodiments, "biodiesel" refers to a fuel product comprised primarily of, or substantially of, fatty acid alkyl esters (e.g., a product having less than about 1%, 0.8%, 0.6%, 0.4%, or less FFAs) derived from the transesterification of a natural oil feedstock with an alcohol. The composition of the fatty acid alkyl esters will depend on the alcohol used in the transesterification reaction. For example, if methanol is the alcohol used in the reaction, the fatty acid alkyl esters will be fatty acid methyl esters (FAME). If ethanol is the alcohol used in the reaction, the fatty acid alkyl esters will be fatty acid ethyl esters (FAEE). Various specification and standards have been established to characterize biodiesel fuels and blend stocks for example, the American Society of Testing and Manufacturing (ASTM) D6751-14 "Standard Specification for Biodiesel Fuel Blend Stock (B100) for Middle Distillate Fuels" which is incorporated herein in its entirety. In alternative embodiments, the biodiesel produced meets or exceeds those specifications established by ASTM D6751-14.

In alternative embodiments, "USP-Grade glycerol" or "food-grade glycerol" is a glycerol product meeting or exceeding the standards set forth by the U.S. Pharmacopeial Convention (USP) for classification as a "USP-grade" glycerol. In alternative embodiments, the systems and methods as provided herein result in the production of USP-grade glycerol as a co-product to the production of biodiesel.

In alternative embodiments, a "co-solvent" is a product that increases the solvolysis activity of the reaction mixture, thereby enabling a more complete conversion of lipids to biodiesel, or a fuel product comprising substantially fatty acid alkyl esters, and increasing the overall yields of the process. In alternative embodiments, the disclosed processes comprise the addition of a co-solvent to the reaction mixture (the mixture of the alcohol and the feedstock). The co-solvent can be, for example, a hydrocarbon or hydrocarbon mixture, or carbon dioxide (CO₂). In the disclosure that follows, CO₂ is the co-solvent used, although those skilled in the art will appreciate that other co-solvents may be substituted in alternative embodiments.

### Processes - a corn oil feedstock (not part of the present invention)

A continuous process is provided for the production of biodiesel and glycerol from a natural oil feedstock comprising high levels of FFAs, e.g. corn oil, such as oil from distiller's corn oil. Methods and processes as provided herein do not include the use of a catalyst in the transesterification reaction; and in alternative embodiments result in a ASTM "B100" grade biodiesel and/or a USP-grade glycerol co-product.

A corn oil feedstock comprising FFAs in the amount of approximately 10% wt by weight of the feedstock, e.g. about 15% FFAs, is combined with methanol that is essentially or substantially free of any contaminates, e.g. about 99.0% methanol, to form a reaction mixture. The molar ratio of methanol to oil in the reaction mixture can be between about 5:1 to about 70:1, e.g. about 20:1, 30:1, 40:1, 50:1 or 60:1. Once the alcohol and feedstock are combined, they are subjected to mixing, e.g. mechanical sheer and or sonication mixing, or equivalents, to form an emulsion. The feedstock and alcohol can be mixed (or equivalent) for between about 5 to about 180 minutes, e.g. about 40, 50, 60, 70, 80 or 90 or more minutes or however much time is needed to form an emulsion. If sonication is selected as the method of mixing, the frequency range can be between about 20 to 100 kHz, e.g. about 42 kHz.

The emulsified reaction mixture is then pumped into reactor comprising a series of concentric metal heat exchangers via a positive displacement pump (or other suitable pump type) wherein the pressure exerted on the reaction mixture is between about 34 bar (500 psig) to about 345 bar (5000 psig), e.g. about 138 bar (2000 psig), as measured at the discharge of the pump. Directly after the discharge of the high-pressure pump, a co-solvent and/or additional FFAs (e.g., FFAs of different structure as in the initial mix) may be added to the reaction mixture, e.g., via a port or equivalent that is operationally connected to the discharge area of the pump. The co-solvent-to-alcohol molar ratio can be between about 0.01:1 to about 5:1, e.g. about 0.12:1. The FFA-to-reaction solution weight ratio can be between about 0.01:1 to 10:1 or about 0.3:1.

The pressurized reaction mixture, comprising the feedstock, alcohol, and the optional co-solvent and/or FFAs are then heated to a temperature in the range of between about 200°C to about 400°C, e.g. 290°C. The reaction mixture is maintained at the desired temperature and pressure and allowed to react for between about 1 minute to about 300 minutes, e.g. about 40, 50, 60, 70, 80 or 90 or more minutes. During the reaction, the supercritical methanol undergoes a transesterification reaction with any triglycerides present in the feedstock to yield FAME and glycerol. Substantially all of the FFAs present in the feedstock undergo an esterification reaction with the alcohol to form FAME and substantially all of the esters in the feedstock, e.g. lipids, phospholipids or other esters, will similarly be subjected to esterification or transesterification for yield FAME.

The resulting product mixture will comprise FAME, water, unreacted methanol, glycerol, co-solvent (if present in the reaction mixture) and possibly other products.

Following the reaction, the product mixture (i.e. the product mixture in which the organic acids are substantially esterified and the esters are substantially transesterified) is discharged from the reactor, e.g., via a high-pressure pump or equivalent, and passed through a heat exchanger, e.g., a high pressure concentric heat exchanger (wherein the pressure is maintained at the level of the reactor), wherein the heat is withdrawn from the product mixture and optionally recovered (where the heat can be recycled for use elsewhere in the process, e.g. to heat the reactor, thereby decreasing the overall energy requirements of the system). In alternative embodiments, the solution then passes through a back-pressure regulator device at a temperature of between about 125°C to about 350°C, or between about 150°C to about 300°C, e.g. about 240°C.

Following the heat recovery step, the product mixture undergoes a flash process wherein the product mixture is transferred to a flash drum or appropriate or equivalent vessel wherein the pressure is reduced from the pressure within the heat exchanger, e.g. above 81 bar (1171 psig) or about 83 bar (1200 psig), to, for example, about atmospheric pressure, or about less than 1 bar (14 psig), e.g. less than 0.07 bar (1 psig), or about 0.007 bar (0.1 psig). The decrease in pressure results in an environment in which the vapor pressure of the methanol exceeds its external pressure (the pressure of the flash drum or vessel), allowing for the methanol, co-solvent (if present) and water (collectively referred to as "the solvent" in this and subsequent steps) to vaporize or "flash" out of the product mixture.

A flash at 0.007 bar (0.1 psig) results in approximately 95% of the solvent present in the product mixture to vaporize and leave the flash vessel, with approximately 5% of the solvent remaining in a liquid state and exiting the bottom of the flash unit along with the remaining products in the product mixture (i.e. the "biodiesel stream"). In such embodiments, the concentration of solvent (i.e. methanol/solvent/water) leaving the flash unit in a liquid state (in the ester stream) is approximately 2 wt.% of the ester stream.

The biodiesel stream (comprising FAME and glycerol, as well as the water and alcohol that was not separated in the previous flash step) leaves the flash unit at a temperature in the range of between about 110 to about 125°C, e.g. 115°C and is sent to a heat exchanger, e.g. a standard shell and tube heat exchanger, wherein it is cooled to about 95°C. The recovered heat can be recycled for use in the process, e.g. to heat the reactor.

The solvent mixture (the methanol/water/ and, if present, co-solvent mixture obtained from the previous flash separation step), wherein the mixture is approximately 95 wt % methanol or 95 wt % methanol/co-solvent (if co-solvent is present) and approximately 5 wt % water is then distilled to yield a substantially pure methanol, e.g., methanol product, e.g. approximately 99.8% or more methanol. The substantially pure methanol product can be recycled to the methanol supply tank for use in subsequent reactions. If present the co-solvent is distilled in the same distillation step to yield a substantially pure co-solvent product, e.g. 99.8% co-solvent. The substantially pure co-solvent can be recycled to for use in subsequent reactions.

After the biodiesel stream is cooled via the heat exchanger, it is transferred to an inline static mixer wherein it is mixed with soft water in a ratio of about 50:1 biodiesel stream-to-water by mass, or in a ratio of 1 g water-to-glycerol by mass. The water and biodiesel stream mixture is then transferred to a decanter wherein a biodiesel stream and an aqueous stream are formed and are separated.

The aqueous stream leaves the decanter comprises methanol, water (including water that was not removed in the flash separation step and water introduced in the present glycerol recovery/water-wash step) and glycerol, is then transferred to a glycerol stripping column, e.g. a 4-stage stripping column or a 6-stage stripping column, in which the aqueous stream is introduced to the top of the column and, upon contacting the bottom of the column is heated such that a vapor phase, comprising primarily methanol and water, is generated and rises to the top of the column where it is removed. The column "bottoms" are a primarily a glycerol product in the range about 85 to about 99.9 wt % glycerol, e.g. about 99.5% glycerol, which can be marketed directly as "splitter crude" grade glycerol or upgraded through techniques known in the art to a USP grade tech glycerol.

The contents of the separated vapor phase comprising water and methanol will vary depending the composition of the feedstock. In one embodiment, e.g., in which corn oil feedstock, the water/methanol product is approximately 55% methanol and 45% water. The alcohol (e.g. methanol)/water product is sent to the alcohol recovery unit wherein it is distilled to yield a substantially pure alcohol, e.g., methanol, product.

The biodiesel stream separated from the decanter is then heated to between about 150°C to about 220°C via a shell-and-tube heat exchanger and is allowed to flash at an absolute pressure in the range of between about 0 bar (0 psig) to about 0.7 bar (10 psig), e.g. 0.07 bar (1 psig). In this flash step, substantially any excess water contained in the biodiesel stream from the decanting step is removed, thereby "drying" the biodiesel fraction in order to meet the water content specifications for ASTM B100 biodiesel. A portion of the FAME (e.g. <5%) in the flash process stream is evaporated with the water in the flash/dryer unit. This material is condensed in a shell-and-tube condenser and is routed back to the process fluid while the temperature is regulated below the methanol/water vapor dew point. In so doing, it remains as a vapor and is routed out of the system.

The "bottoms" of this flash/drying unit are then sent to a distillation column wherein any other contaminates produced during the transesterification reaction, e.g. waxes, unreacted lipids, FFAs, tocopherols, or sterols, or the like, are separated from the FAME to yield a distillate stream comprising ASTM B100-grade biodiesel. The "bottoms" of the distillation column in the present step are then sent to a high vacuum WFE wherein an recovered, unreacted lipids are evaporated and subsequently sent back to the beginning of the process to be combined with the corn oil feedstock for use in subsequent reactions. Alternatively, the material is sent to an additional distillation column where pure streams of tocopherols, sterols, waxes, esters, and FFAs are collected.

### Exemplary 12-Stage Processes

In alternative embodiments, provided are processes for the economically efficient preparation of high-quality biodiesel. In alternative embodiments, provided are systems and processes for the production of high-purity biodiesel, e.g. a biodiesel meeting or exceeding the specifications for B100 biodiesel set forth in ASTM Specification D6751-14,.

In alternative embodiments, the process is a 2-stage process comprising a first hydrolysis stage and a second esterification stage. In alternative embodiments, natural oil feedstocks with high free (un-esterified) fatty acid content are subjected to a first hydrolysis reaction comprising mixing or contacting the natural oil feedstock with water and allowing the mixture to react at a temperature and a pressure below the critical temperature and pressure of water (i.e. below about 374°C and about 221 bar (3200 psig) to generate a reaction product mixture comprising free fatty acids (FFAs), separating or isolating the generated free fatty acids, mixing or contacting the separated or isolated free fatty acids with an alcohol and a co-solvent and allowing the mixture to react at a temperature and a pressure above the critical temperature and pressure of the selected alcohol, thereby causing the free fatty acids to undergo an esterification reaction with the alcohol to generate fatty acid alkyl esters.

In alternative embodiments, the first stage of the process comprises a subcritical water reaction wherein the feedstock is mixed with water and allowed to react at a temperature between about the boiling point of the water at atmospheric pressure (about 1 bar, 14.7 psig), i.e. about 100 °C and the critical temperature of water, i.e. about 374°C, and wherein the pressure of the reaction is sufficient to maintain the water in a liquid state (i.e., at a pressure equal to or greater than the vapor pressure of the water at the specified reaction temperature). In alternative embodiments, the systems and processes as provided herein are more economical and efficient than currently used approaches for the generation of biodiesel from natural oils.

In alternative embodiments, a "feedstock" is the starting material of a process or method as provided herein; and in alternative embodiments, noting that process and method embodiments as provided herein are not limited by any particular mechanism of action, a "feedstock" is a starting material of a process or method as provided herein that undergoes a first hydrolysis reaction to form a first product mixture and a second esterification/transesterification reaction to form a product mixture. In alternative embodiments, the feedstock comprises at least about 1% wt/wt free organic acids, e.g. free fatty acids (hereafter referred to as free fatty acids and abbreviated FFAs), e.g. between about 1% wt/wt FFAs to about 100%, or between about 10% wt/wt FFAs to about 100% wt/wt FFAs, or between about 15% wt/wt to about 25% wt/wt FFAs. In alternative embodiments, the process is feedstock-flexible and is not limited by the ester or free fatty acid content of the feedstock. In certain embodiments, the feedstock used in the process is comprised of 100% esters, e.g. 100% triglycerides or a combination of any of mono- di- and triglycerides in any amount, or the feedstock used in the process is substantially esters. In alternative embodiments, in addition to the FFAs, the feedstock is comprised primarily of, or substantially comprises, triglycerides with carboxylic acid moieties containing between 6 and 28 carbon atoms. In alternative embodiments, the feedstock further comprises smaller amounts of phospholipids, mono- and di-glycerides with carboxylic acid moieties containing between 6 and 28 carbon atoms, and/or non-ester components e.g. waxes, sterols, tocopherols, hydrocarbons and the like.

In alternative embodiments, the natural oil feedstock is "crude" or "unrefined", meaning it has not been treated to remove, for example, free fatty acids, phospholipids (gummed) or other components of the "crude" oil.

In alternative embodiments, a "supercritical alcohol" is an alcohol at or above the critical temperature and pressure of the alcohol. At or above the critical point of the alcohol, distinct liquid and gas phases do not exist, and the phase-boundary between liquid and gas is terminated. Different alcohols have distinct critical temperatures and pressures. For example, methanol is supercritical at or above a temperature of approximately 240°C and pressure of approximately 81 bar (1173 psig), or equivalents. Ethanol is supercritical at or above a temperature of approximately 241°C and pressure of approximately 61 bar (890 psig), or equivalents.

In alternative embodiments, the alcohol used in second stage of the reaction, i.e. the esterification stage, contains between 1 and 5 carbons, or 1, 2, 3, 4, 5 or 6 or more carbons, e.g. methanol, ethanol, propanol, butanol, isobutanol, isopropyl alcohol or a combination thereof. In various other embodiments, conditions may be such that a higher alcohol containing more than 5 carbons are used. In a description alternative embodiments as provided herein, methanol is used as the alcohol; however, in other embodiments other alcohols can be used, and those skilled in the art would understand that other alcohols, e.g., a higher alcohol, can be used.

"biodiesel" refers to a fuel product comprised primarily or substantially of fatty acid alkyl esters derived from the esterification and/or transesterification of a natural oil feedstock with an alcohol. The composition of the fatty acid alkyl esters generated in the disclosed process will depend on the alcohol used in the esterification/transesterification reaction of the second stage of the process. For example, if methanol is the alcohol used in the second stage of the process, the fatty acid alkyl esters will be fatty acid methyl esters (FAME) and the process will generate a biodiesel product comprising FAME. If ethanol is the alcohol used in the reaction, the fatty acid alkyl esters will be fatty acid ethyl esters (FAEE) and the process will generate a biodiesel product comprising FAEE. Various specification and standards have been established to characterize biodiesel fuels and blend stocks for example, the American Society of Testing and Manufacturing (ASTM) D6751-14 "Standard Specification for Biodiesel Fuel Blend Stock (B100) for Middle Distillate Fuels" which is incorporated herein in its entirety. In alternative embodiments, the biodiesel generated in the disclosed process meets or exceeds those specifications established by ASTM D6751-14.

"USP-Grade glycerol" or "food-grade glycerol" is a glycerol product meeting or exceeding the standards set forth by the U.S. Pharmacopeial Convention (USP) for classification as a "USP-grade" glycerol. In alternative embodiments, the systems and methods as provided herein result in the production of USP-grade glycerol as a co-product to the production of biodiesel.

In alternative embodiments, a "co-solvent" is a product or compound that increases the solvolysis activity of the reaction mixture, thereby enabling a more complete conversion and/or a faster reaction of lipids to biodiesel, and in some embodiments increasing the overall yields of the process. In alternative embodiments, processes and methods as provided herein comprise the addition of a co-solvent to the reaction mixture (the mixture of the alcohol and the feedstock). The co-solvent can be, for example, a hydrocarbon or hydrocarbon mixture, or a carbon dioxide (CO₂). In alternative embodiments provided herein, CO₂ is the co-solvent used, however, in other embodiments other co-solvents can be used, and those skilled in the art will appreciate that other co-solvents may be substituted in alternative embodiments.

Are herein described but do not form part of the invention a two-stage method or process comprising a first hydrolysis stage and a second esterification stage. In alternative embodiments, in the first stage of the 2-stage process, a natural oil feedstock is mixed with water and transferred to a reaction vessel, or the feedstock and water are transferred to the reaction vessel separately and mixed therein. In alternative embodiments, the reaction vessel comprising the water and feedstock is then heated and pressurized to allow for the water to reach a, for example, "subcritical" or "superheated" state. A "subcritical" or "superheated" water is a water that has been heated to a temperature of above the boiling point of water at atmospheric presser (1 bar (14.7 psig)), or about 100°C and pressurized such that the pressure is sufficient to prevent the water from boiling or sufficient to maintain the water in a liquid state. In alternative embodiments of the first hydrolysis stage of the process, esters, e.g. glycerides (mono, di-, and tri-glycerides), are hydrolyzed to generate glycerol and free fatty acids. In alternative embodiments of the hydrolysis reaction, triglycerides are hydrolyzed to generate 1 molecule of glycerol and 3 molecules of free fatty acids; di-glycerides are hydrolyzed to generate 1 molecule of glycerol and 2 molecules of free fatty acids; mono-glycerides are hydrolyzed to generate 1 molecule of glycerol and 1 free fatty acid molecule.

An exemplary embodiment of the process is illustrated schematically in Fig. 1 not part of the invention.

### Hydrolysis Reaction (Stage 1) not part of the invention

As shown in process **100** In alternative embodiments, prior to the hydrolysis reaction, the feedstock **101** is first mixed water **102,** e.g. tap water or deionized water in a molar ratio of between about 3:1 to about 100:1 water-to-oil, e.g. between about 10:1 to about 90:1, about 20:1 to about 80:1, about 30:1 to about 70:1, about 35:1 to about 60:1, about 40:1 to about 50:1, or about 40:1 water-to-oil. Optionally, the water and feedstock can be mixed **103** via mechanical sheer, ultrasonication or equivalents or other suitable technique known in the art to form an emulsion or equivalent. In alternative embodiments, the water/feedstock mixture **104** is then pumped or otherwise transferred into a reaction vessel **105,** e.g. a plug-flow, continuously stirred tank (CSTR), or other suitable reactor. In alternative embodiments, the water/feedstock mixture is pumped into the reaction vessel via a positive displacement pump comprising a backpressure regulator valve operationally connected to the reaction vessel. In such embodiments, the hydraulic force generated by compacting the fluid water/feedstock mixture against a back pressure regulator valve of the pump generates pressures of between about 34 bar (500 psig) to about 345 bar (5000 psig) in the reaction vessel. In alternative embodiments, the pressurized water/feedstock mixture passes through the discharge mechanism and into the reaction vessel wherein the generated pressure is maintained for the duration of the hydrolysis reaction. In alternative embodiments, the vessel is maintained at a pressure of about 138 bar (2000 psig).

In alternative embodiments, co-solvent **106** can be added to the water/feedstock reaction mixture in the first stage of the process. The co-solvent can be mixed at the same time that the water and feedstock are mixed, or added to the pressurized water/feedstock mixture in the reaction vessel via a port, for example a port following the discharge mechanism of the pump. The co-solvent can be, for example, an organic acid, e.g. carbonic acid, a hydrocarbon, e.g. methane, ethane, propane, butane, or pentane, or any combination thereof. The amount of the co-solvent in the reaction mixture (along with the water and feedstock), can be in the amount of between about 0.01:1 to 10:1 co-solvent-to-water, e.g. between about 0.05:1 to about 8:1, about 0.1:1 to about 6:1, about 0.15:1 to about 4:1, or about 0.2:1 to about 2:1, or about 0.2:1 co-solvent-to-water.

In alternative embodiments, the reaction vessel **105** comprising the hydrolysis reaction mixture, comprising water, feedstock and, optionally the co-solvent, is the heated to a temperature of between about 150 °C to about 450 °C, e.g. between about 200 °C to about 400 °C, about 250 °C to about 350 °C, or about 300 °C. In one embodiment, the pressure in the reaction vessel is maintained at about 138 bar (2000 psig) and heated to a temperature of about 300 °C, thereby causing the water in the hydrolysis reaction mixture to become a "hot compressed liquid", i.e. a liquid that has been heated to above its atmospheric boiling point (the point at which the liquid boils at atmospheric pressure) and pressurized such that the pressure exceeds the vapor pressure of the liquid thereby causing it to remain in a liquid state.

In alternative embodiments, the contents of the reaction vessel are allowed to react at the selected temperature and pressure for a period of between about 1 to about 300 minutes, e.g. about 2 to about 250 minutes, about 4 to about 200 minutes, about 6 to about 150 minutes, about 8 to about 100 minutes, about 10 to about 90 minutes, about 12 to about 70 minutes, about 14 to about 50 minutes, about 16 to about 40 minutes, about 18 minutes to about 30 minutes, or about 20 minutes, or until substantially all, or most ( 70% or more of the ester bonds, e.g. 75%, 80%, 90%, 95%, 97%, 98%, 99% or more) of the ester bonds in the feedstock have been hydrolyzed, thereby "cleaving" or separating, via hydrolysis acting at the ester bonds of the esters in the feedstock, fatty acid molecules to generate "free" (un-esterified) fatty acids.

In alternative embodiments, after the hydrolysis reaction mixture (feedstock, water, and optionally a co-solvent) has been reacted for the desired period of time (e.g., after substantially hydrolyzing all fatty acid molecules to generate "free" (un-esterified) fatty acids), the resulting "hydrolysis product mixture" **107** will vary depending on the composition of the feedstock, but may comprise, for example, free fatty acids, glycerol, water, unsaponifiable material (e.g. waxes, sterols and hydrocarbons if present in the feedstock), and glycerol phosphatidyls (resulting from the cleaving of the free fatty acids from phospholipids if phospholipids are present in the feedstock), as well as any unreacted (un-hydrolyzed) esters e.g. glycerides, and phospholipids.

In alternative embodiments, a heat-recovery unit operation is included in the process wherein, following the hydrolysis reaction, incoming hydrolysis reaction mixture material (feedstock, water and optionally a co-solvent) is heated with the heat contained in the hydrolysis product mixture using a heat-exchanger device, e.g. a shell-and-tube heat exchanger or other suitable heat recovery system. In alternative embodiments, a shell-and-tube heat exchanger is utilized and comprises an outer cylindrical tube or "shell" having an exterior wall and an interior wall defining an internal cavity within which one or more tubes are contained, each having a smaller diameter than the outer tube, and each having an exterior wall and an interior wall defining an internal cavity.

In an exemplary embodiment, a shell-and-tube heat exchanger is utilized in the process and the heated material (the hydrolysis product mixture), flows within the "tube" portion (within the interior cavity of the tubes contained within the shell) of the shell-and-tube heat exchanger and the incoming process material, having just exited the discharge of the high-pressure pump and therefore pressurized to the desired pressure of the hydrolysis reaction, flows counter-currently within the "shell" of the shell-and-tube heat exchanger, (between the exterior walls of the tubes contained within the shell and the interior wall of the shell). Heat is thereby transferred and simultaneously heats the incoming reaction mixture and cools the hydrolysis product mixture.

The temperature of the hydrolysis product mixture can be decreased from the temperature of the hydrolysis reaction by, for example, between about 70°C and about 370°C, depending on the temperature of the hydrolysis reaction and the desired temperature of the product mixture in subsequent unit operations. In certain embodiments, the temperature of the reaction vessel is maintained at a temperature of about 200°C during the hydrolysis reaction and the hydrolysis product mixture is cooled to a temperature of about 120°C in the foregoing eat exchange step, a reduction in temperature of about 80°C. In other embodiments, the hydrolysis reaction is conducted at higher or lower temperatures and the hydrolysis reaction products are cooled to higher or lower temperatures than about 120°C in the heat exchange step.

In alternative embodiments, following the heat-exchange, the pressure of the cooled hydrolysis product mixture is reduced by, for example, passing the reaction products through a backpressure regulator device or equivalent **108** that decreases the pressure of the product mixture to about atmospheric pressure (i.e. 1 bar, 14.7 psig). In alternative embodiments, the pressure of the hydrolysis reaction products is decreased rapidly and a portion of the water in the product mixture "flashes" off, i.e. vaporizes, as the pressure exerted on the reaction products is reduced to below the vapor pressure of the cooled mixture. Any suitable vessel known in the art may be used for this step and is therefore not limited by a specific apparatus or device. The flashed water **109** can be captured and recycled in the process for subsequent hydrolysis reactions.

In alternative embodiments, the hydrolysis product mixture, following the optional flash step above **110,** is further cooled to a temperature of between about 70°C and about 110°C, e.g. between about 80°C and about 105°C, between about 90°C and about 100°C, or about 90°C. This cooling step is optionally achieved via the use of a heat exchanger, thereby allowing for the recovery of heat, which can be recycled for use elsewhere in the process.

In alternative embodiments, the product mixture is then transferred to an "oil/water separation unit" **111** e.g. a centrifuge, decanter, hydrocyclone (or series of hydrocyclones), or other suitable apparatus or system wherein the product mixture is separated into a lipid phase **112** and an aqueous phase **113,** and the lipid and aqueous phases are physically separated from one another thereby generating two separate streams for further processing. In alternative embodiments, the lipid phase **112** comprises the free fatty acids and possibly other lipids (if all of the ester bonds in the feedstock was not completely hydrolyzed) e.g. glycerides and phospholipids, and an aqueous phase **113** comprising water and glycerol and, if phospholipids were present in the feedstock, glycerol phosphatidyls. In alternative embodiments, the lipid phase **112** floats on top of the aqueous phase **113** due to the differences in density of the products within each phase and the lipid phase is removed from the aqueous phase.

In alternative embodiments, the separated lipid phase **113** is subjected to an optional "drying" step **114** wherein any water **115** that was entrained in the lipid during the lipid phase separation step is removed from the remaining lipid products (e.g. free fatty acids and glycerides), thereby generating a lipid product **116** substantially free of water. In alternative embodiments, the drying is achieved by heating the lipid phase to a temperature of between about 40°C and about 200°C, e.g. between about 100°C and about 195°C, about 120°C and about 190°C, about 140°C and about 185°C, or about 185°C under a vacuum of between about 0.007 bar (about 5 Torr) to about 1 bar (about 770 Torr absolute), e.g. between about 0.013 bar (about 10 Torr) and about 0.8 bar (about 600 Torr absolute), between about 0.02 bar (about 15 Torr) and about 0.67 bar (about 500 Torr absolute), between about 0.027 bar (about 20 Torr) and about 0.53 bar (about 400 Torr absolute), between about 0.04 bar (about 30 Torr) and about 0.4 bar (about 300 Torr absolute), between about 0.05 bar (about 35 Torr) and about 0.27 bar (about 200 Torr absolute), between about 0.053 bar (about 40 Torr) and about 0.133 bar (about 100 Torr absolute), between about 0.06 bar (about 45 Torr) and about 0.1 bar (about 80 Torr absolute), between about 0.07 bar (about 50 Torr) and about 0.08 bar (about 60 Torr absolute), or about 0.075 bar (about 55 Torr absolute). The water that has been removed from the lipid phase can optionally be recycled in the process.

In alternative embodiments, the aqueous phase **113** generated in the lipid separation step is transferred to a distillation column, stripping column, or other suitable separation column or device **117,** wherein the glycerol is separated from the remaining products in the aqueous phase. The configuration of the column (e.g. the stripping column or distillation column) can vary depending on the desired product output and composition of the aqueous phase that is the input stream to the column. In alternative embodiments, the distillation column is a packed distillation column. In other embodiments, the distillation column is a trayed distillation column comprising between 1 and 50 stages, e.g. between 2 and 40 stages, between 3 and 30 stages, between 4 and 20 stages, between 5 and 10 stages, or 6 stages.

In alternative embodiments, the aqueous phase is transferred to a glycerol distillation column, e.g. a 6-stage distillation column, in which the aqueous stream is introduced into the column and is heated such that a vapor phase, comprising primarily water, or water and alcohol (if the input to the glycerol distillation unit includes the glycerol-containing aqueous phase generated in the second stage of the process), is generated and rises to the top of the column where it is removed. In this exemplary embodiment, the column "bottoms" are a primarily a glycerol product **118** in the range about 85 to about 99.9 wt % glycerol, e.g. about 99.5% glycerol, which can be marketed directly as "splitter crude" grade glycerol or upgraded through techniques known in the art to a USP grade tech glycerol. The aqueous phase is distilled under a vacuum of between about 0.013 bar (about 10 torr) and 1 bar (770 Torr absolute), e.g. between about 0.067 bar (about 50 torr) and about 0.67 bar (about 500 Torr absolute), about 0.13 bar (about 100 Torr) and about 0.53 bar (about 400 Torr absolute), about 0.27 bar (about 200 Torr) and about 0.4 bar (about 300 Torr absolute), or about 0.33 bar (about 250 Torr absolute). The distillate stream generated in the distillation column is deionized water **119,** which can be recycled in in the process for use in subsequent hydrolysis reactions.

### Esterification Reaction of the invention

The lipid phase **116** generated in the foregoing lipid separation step following the hydrolysis reaction and comprising free fatty acids (FFAs), and possibly esters e.g. glycerides and/or phospholipids referred to herein as the "esterification feedstock" is combined with an alcohol **120,** e.g. methanol or ethanol, that is essentially free of any contaminants, e.g. about 99.0% alcohol, to form a reaction mixture **121.** In certain embodiments, an alcohol with lower purity may be used, e.g. an alcohol comprising about 95% alcohol and 5% water. Lower-purity alcohols are generally cheaper than high-purity alcohols and there use may therefore result in more favorable economics despite lower FFA yields from the process. The lipid phase generated in the first stage of the process **116** is therefore the feedstock for the second stage of the process. The molar ratio of the alcohol to the esterification feedstock in the reaction mixture **121** can be between about 5:1 to about 70:1, e.g. about 40:1. In alternative embodiments, the moisture content (amount of water) of the esterification feedstock, is between about 0 and 5% by weight of the feedstock. Once the esterification feedstock, and alcohol are combined, they are optionally mixed, e.g. via mechanical sheer and or sonication mixing, to form an emulsion or equivalent. The esterification feedstock and alcohol can be mixed for between about 5 to about 180 minutes, e.g. about 60 minutes or an emulsion is formed. If sonication is selected as the method of mixing, the frequency range can be between about 20-100 kHz, e.g. about 42 kHz. The combined and optionally emulsified esterification feedstock and alcohol mixture is referred to herein as the "esterification reaction mixture."

In alternative embodiments, the esterification reaction mixture **121** is then pumped into a reactor **122** comprising a series of heat exchangers, e.g., concentric metal heat exchangers, via a positive displacement pump (or other suitable pump type) wherein the pressure created from pumping the mixture against a backpressure regulator valve on the reaction mixture is between about 34 bar (500 psig) to about 345 bar (5000 psig), e.g. about 138 bar (2000 psig), as measured at the discharge of the pump. Directly after the discharge of the high-pressure pump, a co-solvent **123,** e.g. an organic acid or a hydrocarbon e.g. methane, ethane, propane, butane, or pentane or any combination thereof, may optionally be added to the esterification reaction mixture via a port that is operationally connected to the discharge area of the pump. The amount of optional co-solvent-to-alcohol in the esterification reaction mixture can be, for example a molar ratio of between about 0.01:1 to about 5:1, e.g. about e.g. between about 0.05: 1 to about 8:1, about 0.1: 1 to about 6:1, about 0.15: 1 to about 4:1, or about 0.2:1 to about 2:1, or about 0.2:1 co-solvent-to-alcohol.

In alternative embodiments, the pressurized esterification reaction mixture, comprising the esterification feedstock, alcohol, and the optional co-solvent and/or FFAs are then heated in a suitable reaction vessel **122** to a temperature in the range of between about 200°C to about 400°C, e.g. 290°C, or a temperature about the critical temperature of the selected alcohol. In an exemplary embodiment, the alcohol in the esterification reaction mixture is methanol and the temperature of the reaction is above the critical temperature of methanol, i.e., above about 240°C, e.g. about 300°C, and the pressure is above the critical pressure of the methanol, i.e. about 81 bar (1174 psig). The esterification reaction mixture is maintained at the desired temperature and pressure and allowed to react for between about 1 minute to about 300 minutes, e.g. between about 5 minutes about 60 minutes, about 10 minutes and about 40 minutes, or about 15 minutes about 25 minutes, or about 20 minutes. During the reaction, the alcohol esterifies the free fatty acids to generate fatty acid alkyl esters, e.g. fatty acid methyl esters (FAME) if methanol is the alcohol used in the reaction. The alcohol undergoes a transesterification reaction with the esters (if present) in the reaction mixture to generate fatty acid alkyl esters. In alternative embodiments, substantially all of the FFAs present in the feedstock undergo an esterification reaction with the alcohol to generate fatty acid alkyl esters and substantially all of the esters in the feedstock, e.g. glycerides, phospholipids or other esters, will similarly be subjected to transesterification to generate fatty acid alkyl esters.

If water is present in the esterification reaction mixture, the water can allow for less severe reaction conditions, e.g. lower temperatures and pressures, by increasing the solvolysis activity of the mixture, relative to a mixture comprising alcohol and the esterification feedstock alone i.e. without water. The water can also react with a portion of the ester bonds present in the esterification feedstock, thereby hydrolyzing a portion of the esters to generate free fatty acids. The hydrolysis of esters by water can allow for increased free fatty acid yield from the esterification reaction with decreased reaction times. In alternative embodiments, during the second stage of the process, the esterification reaction allows for the simultaneous hydrolysis and esterification of esters in the esterification feedstock. As an example, a triglyceride in the esterification feedstock may be subjected to hydrolysis with water to generate one molecule of glycerol and 3 molecules of free fatty acids. In the same reaction step, the generated 3 free fatty acids molecules can undergo an esterification reaction with the alcohol in the esterification reaction mixture to generate three molecules of fatty acid alkyl esters.

The product mixture generated by the esterification reaction, referred to herein as the "esterification product mixture," **124** can comprise fatty acid alkyl esters, water, unreacted alcohol, glycerol, co-solvent (if present in the reaction mixture) and possibly other products, e.g. glycerol phosphatidyls is phospholipids are present in the feedstock. In alternative embodiments the esterification product mixture may also comprise esters that did not undergo a hydrolysis or transesterification reaction and therefore remain "unreacted." The portion of unreacted esters after the esterification reaction can be between about 0.1% to about 20% of the esters that were present in the esterification feedstock, e.g. between about 1 and 15%, about 2 and 10% or about 2% of the esters that were present in the esterification feedstock. The esterification product mixture may also comprise free fatty acids (FFAs) that did not react with the alcohol to generate fatty acid alkyl esters and therefore remain "unreacted." The portion of unreacted free fatty acids after the esterification reaction can be between about 0.1% to about 20% of the free fatty acids that were present in the esterification feedstock, e.g. between about 1 and 15%, about 2 and 10% or about 3% of the free fatty acids that were present in the esterification feedstock.

In alternative embodiments, following the reaction, the esterification product mixture (i.e. the product mixture in which the fatty acids generated in the first hydrolysis stage of the process are substantially esterified and the esters that were not hydrolyzed in the first hydrolysis stage of the process are substantially transesterified) is discharged from the reactor, e.g., via a high-pressure pump, and passed through a heat exchanger, e.g., a high pressure concentric heat exchanger (wherein the pressure is maintained by the backpressure regulator), and wherein the heat is withdrawn from the product mixture and optionally recovered, for example, where the heat is recycled for use elsewhere in the process, e.g. to heat the reactor, thereby decreasing the overall energy requirements of the system. In alternative embodiments, the mixture then passes through a backpressure regulator device at a temperature of between about 125°C to about 350°C, or between about 150°C to about 300°C, e.g. about 240°C.

In alternative embodiments, following the heat recovery step, the esterification product mixture is optionally subjected to a flash separation process wherein the pressure of the cooled esterification product mixture is reduced by, for example, passing the product mixture through a backpressure regulator device and into a flash drum or other appropriate or equivalent vessel **125** wherein the pressure of the product mixture is reduced from the pressure within the heat exchanger (e.g. above about 81 bar (1171 psig) or about 138 bar (2000 psig) to about atmospheric pressure (i.e. about 1 bar, 14.7 psig). In alternative embodiments, the pressure of the esterification product mixture is decreased rapidly and the decrease in pressure. The decrease in pressure results in an environment in which the vapor pressure of the alcohol exceeds its external pressure (the pressure of the flash drum or vessel), allowing for the alcohol, co-solvent (if present) and any water (collectively referred to as "the solvent" **126** in this and subsequent steps) to vaporize or "flash" out of the product mixture.

In alternative embodiments, the optional flash step causes approximately 95% of the solvent present in the product mixture to vaporize and leave the flash vessel, with approximately 5% of the solvent remaining in a liquid state and exiting the bottom of the flash unit along with the remaining products in the product mixture, referred to herein as the "ester stream" **127.** In such embodiments, the concentration of solvent (i.e. alcohol/ and optionally water and/or the co-solvent) leaving the flash unit in a liquid state (in the ester stream **127**) is approximately 2 wt.% of the ester stream.

In alternative embodiments, the ester stream (comprising fatty acid alkyl esters e.g. FAME and glycerol, any unreacted free fatty acids and/or esters e.g. glycerides, as well as the water and alcohol that was not separated in the previous flash step) **127** leaves the flash unit **125** at a temperature in the range of between about 110 to about 125°C, e.g., 115°C and is optionally sent to a heat exchanger, e.g. a standard shell and tube heat exchanger, wherein it is cooled to about 95°C. The recovered heat can be recycled for use in the process, e.g. to heat the esterification reactor 122.

In alternative embodiments, the solvent mixture (the alcohol/water/ and, if present, co-solvent mixture obtained from the previous flash separation step) **126,** wherein the mixture is approximately 95 wt % alcohol or 95 wt % alcohol/co-solvent (if co-solvent is present) and approximately 5 wt % water is then distilled to yield a substantially pure alcohol product, e.g., a substantially pure methanol product, e.g. approximately 99.8% or more alcohol. The distillation unit **128** can comprise, for example, a packed or trayed distillation columns, e.g., a trayed distillation column comprising between 1 and 75 stages, e.g. between 5 and 70 stages, between 10 and 65 stages, between 15 and 60 stages, between 20 and 55 stages, between 25 and 50 stages, or between 30 and 45 stages, e.g. 40 stages. The distillation is achieved under a vacuum of between about 0.34 bar (5 psig) and 1.4 bar (20 psig) e.g. 1 bar (14.7 psig) to generate a substantially pure alcohol product **129.** The generated substantially pure alcohol product **129** can be recycled to the alcohol supply tank for use in subsequent reactions. If present the co-solvent is distilled in the same distillation step to yield a substantially pure co-solvent product, e.g. 99.8% co-solvent. The substantially pure co-solvent can be recycled to for use in subsequent reactions.

In alternative embodiments, after the ester stream **127** is optionally cooled via a heat exchanger, it is transferred to mixing vessel wherein it is mixed with water via, for example, an inline static mixer or wherein it is mixed with soft water in a ratio of about 50:1 ester stream-to-water by mass, or in a ratio of 1 g water-to-glycerol by mass. The water and ester stream mixture is then transferred to a suitable separation vessel **130,** e.g. a decanter, a centrifuge, or a hydrocyclone or series of hydrocyclones, wherein a lipid stream, referred to herein as the "biodiesel stream" **131** and an aqueous phase **132** are formed and are separated.

In alternative embodiments, the aqueous stream **132** that leaves the decanter comprises alcohol, water (including any water that was not removed in the flash separation step and water introduced in the present glycerol recovery/water-wash step) and glycerol, is then transferred to a glycerol stripping column **117,** e.g. a 6-stage stripping column, in which the aqueous stream is introduced to the top of the column and, upon contacting the bottom of the column is heated such that a vapor phase, comprising primarily alcohol and water, is generated and rises to the top of the column where it is removed. In this exemplary embodiment, the column "bottoms" are a primarily a glycerol product in the range about 85 to about 99.9 wt % glycerol, e.g. about 99.5% glycerol, which can be marketed directly as "splitter crude" grade glycerol or upgraded through techniques known in the art to a USP grade tech glycerol. The generated glycerol product can optionally be mixed with the glycerol product generated during the first hydrolysis stage of the process. In alternative embodiments, the aqueous stream generated in the first (hydrolysis) stage of the process comprising glycerol is combined with the aqueous stream generated in the second (esterification) stage of the process and are distilled simultaneously to generate the glycerol product.

In alternative embodiments, the biodiesel stream separated from the decanter is then heated to between about 150 °C to about 220 °C via a shell-and-tube heat exchanger and is allowed to flash at an absolute pressure in the range of between about 0 bar (about 0 psig) to about 0.7 bar (about 10 psig), e.g. 0.07 bar (1 psig), or between about 0.07 bar (about 5 torr) and 1 bar (770 torr), e.g. 0.013 bar (10 torr) to about 0.4 bar (about 300 torr), between 0.027 bar (20 torr) and 0.2 bar (150 torr), between 0.04 bar (30 torr) and 0.133 bar (100 torr), between 0.053 bar (40 torr) and 0.107 bar (80 torr), or about 0.073 bar (about 55 torr). In this flash step **133,** substantially any excess water **134**contained in the biodiesel stream from the decanting step is removed, thereby "drying" the biodiesel fraction in order to meet the water content specifications for ASTM B100 biodiesel, if methanol is the alcohol used in the esterification reaction. In alternative embodiments, a portion of the fatty acid alkyl esters (e.g. less than about 5%) in the flash process stream is evaporated with the water in the flash/dryer unit. This material can be condensed in a shell-and-tube condenser and can be routed back to the process fluid while the temperature is regulated below the methanol/water vapor dew point. In so doing, it remains as a vapor and is routed out of the system.

In alternative embodiments, the "bottoms" **135** of this flash/drying unit are then sent to a distillation column wherein the fatty acid alkyl esters are separated from the other products present in the bottoms, e.g. waxes, unreacted lipids e.g. glycerides, FFAs, tocopherols, or sterols, or the like to yield a distillate stream comprising substantially pure e.g. 98% or more, fatty acid alkyl esters. In alternative embodiments, the distillation column **136** can be, for example a packed distillation column or a trayed distillation column. In alternative embodiments, the distillation column comprises between 1 and 50 stages, e.g. between 5 and 45 stages, between 10 and 40 stages, between 15 and 35 stages, between 20 and 30 stages, or 25 stages. In alternative embodiments, the distillation is conducted under a vacuum in the range of between about 0.01 bar and 0.27 bar (about 1 and 200 Torr absolute), e.g. between about 0.003 and 0.2 bar (about 2 and 150 Torr absolute), between 0.005 and 0.133 bar (4 and 100 Torr absolute), between 0.008 and 0.07 bar (6 and 50 Torr absolute), between 0.01 and 0.027 bar (8 and 20 Torr absolute), or about 0.013 bar (about 10 Torr absolute). In alternative embodiments wherein methanol is the alcohol used in the esterification reaction, the distillate stream comprises substantially pure FAME **137** meeting or exceeding the standards established for ASTM B100-grade biodiesel.

In alternative embodiments, the "bottoms" **138** of the distillation column **136** in the previous fatty acid alkyl ester distillation step can comprise, for example, unreacted esters, e.g. glycerides any combination of mono-glycerides, di-glycerides, and triglycerides), sterols, tocopherols, and various unsaponifiable material e.g. waxes and hydrocarbons. In alternative embodiments, the bottoms are sent to a high vacuum distillation unit **139** wherein any recovered, unreacted lipids, mono-glycerides, and free fatty acids **140** are evaporated and subsequently sent back to the beginning of the process to be combined with the starting feedstock **116** of the second stage of the process for use in subsequent reactions. In alternative embodiments, the temperature of the stream entering the distillation unit in the present step will be between about 180°C to about 280°C, e.g. about 240°C. In alternative embodiments, the mixture is allowed to flash at an absolute pressure between about 0.00001 bar to about 1 bar (about 0.01 to about 770 Torr), e.g. 0.001 bar (1 Torr). During the present distillation step, a distillate stream **140** is generated comprising the FFAs and mono-glycerides (if present in the incoming bottoms material), and a bottoms stream comprising the non-monoglyceride and FFA components of the incoming bottoms stream. The distillate stream comprising the FFAs and any mono-glycerides is optionally passed through a heat exchange unit wherein heat is recovered and recycled in for use in elsewhere in the process.

In alternative embodiments, the distillate stream comprising the FFAs and mono-glycerides is recycled within the second esterification stage of the process and can therefore become a portion of the esterification reaction mixture **121.** In alternative embodiments, the FFA/mono-glyceride stream is combined with the esterification feedstock (i.e. the lipid stream recovered in the first hydrolysis stage of the process comprising the generated free fatty acids and possibly other lipids), alcohol, water and the optionally co-solvent. By recycling the FFA/mono-glyceride stream, the yield of fatty acid alkyl ester generated in the process is increased and is therefore more efficient an economical than other methods in the art for generating fatty acid alkyl esters from natural lipid sources.

Process for conversion of high-FFA feedstocks with high percentages of saturated fatty acids:
In alternative embodiments, provided are systems and processes for the economically efficient preparation of high-quality biodiesel and other products from lipid feedstocks comprising a high percentage (e.g. >10%) of free fatty acids (FFAs), wherein a high percentage of the fatty acids in the feedstock (both in the form of glycerides and FFAs) are saturated, i.e. lack any carbon-carbon double bonds. In alternative embodiments, provided are systems and processes for the production of biodiesel meeting or exceeding the specifications for B100 biodiesel set forth in ASTM Specification D6751-14 from feedstocks comprising high percentages of free fatty acids and wherein a high percentage or substantially most of the fatty acids in the feedstocks are saturated. Natural oil feedstocks with high saturated FFA content, e.g. a palm oil fatty acid distillate (PFAD) are subjected to an esterification/ transesterification reaction with an alcohol under conditions at or above the critical temperature and pressure of the alcohol in the absence of any catalyst, wherein the FFAs in the feedstock undergo an esterification reaction with the alcohol to generate fatty acid alkyl esters and the glycerides undergo a transesterification reaction with the alcohol to generate fatty acid alkyl esters. The product generated in the esterification/transesterification reaction is separated into a "light" fraction comprising the lighter alkyl esters (i.e. alkyl esters with 16 or fewer carbons) and a "heavy" fraction comprising heavy alkyl esters (e.g. alkyl esters with more than 16 carbons) and any unreacted FFAs.

In alternative embodiments, the esterification/transesterification reaction converts approximately 95% or more of the FFAs and glycerides to fatty acid alkyl esters. In order to generate a biodiesel product meeting the specification set forth in relevant industrial standards, e.g. ASTM Specification D6751-14, generated product must be distilled to or otherwise purified to increase the percentage of alkyl esters in the final product. In alternative embodiments, the feedstock comprises high percentage of saturated fatty acids, e.g. 40% or more saturated fatty acids. Unreacted saturated free fatty acids, e.g. palmitic acid, in the esterification/transesterification product have very similar vapor pressures to the lighter alkyl esters, e.g. methyl stearate, making the isolation of a pure (98% or more) alkyl ester product difficult. Alternative embodiments provided herein overcome this problem by separating the lighter alkyl esters generated in the esterification/ transesterification reaction from the "bottoms" comprising the heavier (i.e. longer carbon chains) alkyl esters and unreacted FFAs, and any esters (e.g. glycerides and phospholipids) or other saponifiable material. The bottoms are then subjected to a second reaction or processing step, e.g. a second esterification/transesterification reaction, wherein the majority (e.g. 95% or more) of the unreacted FFAs and esters from the first esterification/transesterification product are converted to alkyl esters. The resulting product is suitably purified to meet the relevant industrial standards for biodiesel and can optionally be combined with the separated lighter alkyl esters separated from the initial esterification/transesterification reaction.

In alternative embodiments, a "feedstock" is the starting material of a process or method as provided herein; and in alternative embodiments, noting that process and method embodiments as provided herein are not limited by any particular mechanism of action, a "feedstock" is a starting material of a process or method as provided herein that undergoes an esterification/transesterification reaction to form a product mixture. In alternative embodiments, the feedstock is comprised of lipids derived from a natural source, e.g., a plant or an animal source, wherein in alternative embodiments the feedstock comprises at least about 10% wt/wt free organic acids, e.g. free fatty acids (hereafter referred to as free fatty acids and abbreviated FFAs), e.g. between about 1% wt/wt FFAs to about 100%, or between about 10% wt/wt FFAs to about 100% wt/wt FFAs, or between about 50% wt/wt to about 80% wt/wt FFAs. The feedstock is a natural oil, or is a processing by-product of a natural oil having a fatty acid profile comprising a high percentage of saturated fatty acids, e.g. palm oil. In alternative embodiments, a high percentage of saturated fatty acids is above about 30%, e.g. between about 40% to 55%. In alternative embodiments, the feedstock is a fatty acid distillate generated during the processing of a natural oil, e.g. a palm oil fatty acid distillate (PFAD) or other fatty acid distillate. In alternative embodiments palm oil is used, e.g., because it has a favorable fatty acid profile due to its high percentage of saturated fatty acids (typically between about 40-50% of the fatty acids in the oil). PFAD typically has a FFA content of about 70% or more and has a fatty acid profile similar or mirroring that of the palm oil from which it was generated (i.e. between about 40% and 55% saturated fatty acids). Table 1 shows the fatty acid profile of a typical palm oil.

**Table 1. Fatty acid composition of palm oil**

| Fatty acid | Fatty acid name | Percentage range (wt% as methyl esters) |
|---|---|---|
| C12:0 | Lauric acid* | 0.0 - 0.5 |
| C14:0 | Myristic acid* | 0.9 - 1.5 |
| C16:0 | Palmitic acid* | 39.2 - 45.8 |
| C16:1 | Palmitoleic acid | 0.0 - 0.4 |
| C18:0 | Stearic acid* | 3.7 - 5.4 |
| C18:1 | Oleic acid | 37.4 - 44.1 |
| C18:2 | Linoleic acid | 8.7 - 12.5 |
| C18:3 | α-Linolenic acid | 0.0 - 0.6 |
| C20:0 | Arachidic acid* | 0.0 - 0.5 |

| | | |
|---|---|---|
| * Saturated fatty acid | | |

As can be seen in table one, the total percentage of saturated fatty acids in a typical palm oil (and therefore in the corresponding palm oil fatty acid distillate) is between about 43.8% and 53.7%. In alternative embodiments, a palm oil fatty acid distillate (PFAD) feedstock comprising FFAs, glycerides (including mono- di- and triglycerides), and optionally other compounds e.g., phospholipids, sterols, vitamin E compounds (tocopherols and/or tocotrienols), squalene, or other compounds present in the oil from which the PFAD feedstock was generated, is reacted with an alcohol at a temperature and a pressure above the critical temperature and pressure of the selected alcohol for a period of time sufficient to allow for the esterification of the majority (e.g. about 95%) of the FFAs in the feedstock and for the transesterification of the majority (e.g. about 95%) of the esters (e.g. mono- di- and triglycerides and phospholipids). In the foregoing reaction, a reaction product is generated wherein the majority of the FFAs and the majority of the esters present in the feedstock have been converted to fatty acid alkyl esters. In alternative embodiments, the reaction product also comprises glycerol (generated during the transesterification of the glycerides and phospholipids in the feedstock), unreacted FFAs (e.g. about 5% of the total amount of FFAs in the feedstock prior to the reaction), unreacted glycerides, and optionally other compounds e.g. unreacted phospholipids, sterols, vitamin E compounds (tocopherols and/or tocotrienols), squalene, and/or other unsaponifiable material.

In alternative embodiments, after the esterification/transesterification reaction, the reaction product, wherein approximately 95% or more of the FFAs and glycerides have been converted to fatty acid alkyl esters, has a fatty acid alkyl ester profile corresponding to the fatty acid profile of the feedstock used in the esterification/ transesterification reaction. For example, if a PFAD is the feedstock, the reaction product resulting from the esterification/transesterification reaction will be comprised of between about 40% and 55% saturated fatty acid alkyl esters, the majority of which will be alkyl palmitate (e.g. methyl palmitate if methanol is the alcohol used in the esterification/transesterification reaction). In order to generate a biodiesel product of sufficient purity (i.e. sufficiently low FFA, glycerol, and saponifiable content or a biodiesel product meeting the requirements of ASTM D6751-12 for B100 biodiesel), the reaction product generated in the esterification/transesterification reaction must be processed to generate a purified fatty acid alkyl ester product, e.g. a fatty acid alkyl ester product comprising 98% or more fatty acid alkyl esters. In alternative embodiment, the purification or separation of the substantially pure fatty acid alkyl ester product is achieved using any suitable technique known in the art, e.g. distillation. Unreacted, saturated FFAs in the reaction product have similar vapor pressures to heavier fatty acid alkyl esters, i.e. fatty acid alkyl esters with more than 16 carbons, making separation via distillation difficult. If PFAD is the feedstock used in the reaction, the majority of unreacted FFAs in the esterification/transesterification reaction product will be palmitic acid. Because palmitic acid is fully saturated, it has a similar vapor pressure to longer-chain fatty acid alkyl esters e.g. methyl stearate.

In order to generate a substantially pure biodiesel product from a feedstock having a fatty acid profile comprising a high percentage of saturated fatty acids, and comprising a high percentage of FFAs, e.g. a palm oil fatty acid distillate (PFAD), the esterification/transesterification reaction product is distilled, e.g., in a conventional distillation column or equivalent, to separate the lighter fatty acid alkyl esters (e.g. those fatty acid alkyl esters with 16 or fewer carbons) from the other components of the reaction product. If PFAD is the feedstock, the majority of the fatty acid alkyl esters will be alkyl esters of palmitic acid (e.g. methyl palmitate if methanol is the alcohol used in the reaction), as the majority of the fatty acids present in the feedstock with 16 or fewer carbons will be palmitic acid. The "bottoms" in the distillation column will be heavy fatty acid alkyl esters (alkyl esters with more than 16 carbons), unreacted FFAs, any unreacted esters e.g. mono- di- and triglycerides, phospholipids, and any other unsaponifiable material in the feedstock e.g. sterols, vitamin E compounds (tocopherols and/or tocotrienols), squalene, or other compounds.

In alternative embodiments, the bottoms of the distillation column are then subjected to a second esterification/transesterification reaction with a supercritical alcohol as described above wherein approximately 95% of the unreacted FFAs and esters from the first esterification/transesterification reaction are converted to fatty acid alkyl esters. In alternative embodiments, the product mixture generated in the second esterification/ transesterification reaction generates a product mixture comprising less than about 1% FFA. In alternative embodiments, the second product mixture is processed to separate the fatty acid alkyl esters from the remaining components of the product mixture using, for example, distillation to generate an alkyl ester product that is suitable for use as an ASTM B 100 biodiesel. The alkyl ester biodiesel product separated in the second distillation or other separation technique can optionally be combined with the alkyl esters separated from the first reaction product to increase the overall biodiesel yield of the process.

In alternative embodiments, the bottoms of the distillation column undergo an acid-catalyzed alcohol esterification reaction (instead of a second esterification/ transesterification reaction with a supercritical alcohol) comprising a strong acid cation exchange resin. In this configuration, the reaction is an alcohol esterification reaction in the presence of a strong acid cation resin, the resin acting as an acid catalyst of the reaction. In alternative embodiments wherein the bottoms of the distillation column are subjected to an acid-catalyzed alcohol esterification reaction, the bottoms, comprising the unreacted FFAs, any unreacted esters e.g. mono- di- and triglycerides, phospholipids, and any other unsaponifiable material in the feedstock e.g. sterols, vitamin E compounds (tocopherols and/or tocotrienols), squalene, or other compounds, is mixed with an alcohol e.g., methanol to form an alcohol/bottoms mixture. In alternative embodiments, the alcohol/bottoms mixture is heated using, for example, a heat exchanger, e.g., a heat exchanger operationally connected to another portion of the process, to between about 80 and 100 °C and passed through a pipe or other suitable container or vessel comprised a packed cation resin. In alternative embodiments, the mixture is pumped through the resin until substantially all of the saponifiable material in the mixture is converted to fatty acid alkyl esters. In alternative embodiments, following the reaction, any unreacted alcohol is flashed off and recovered and recycled in the process.

In alternative embodiments, a "supercritical alcohol" is an alcohol at or above the critical temperature and pressure of the alcohol. At or above the critical point of the alcohol, distinct liquid and gas phases do not exist, and the phase-boundary between liquid and gas is terminated. Different alcohols have distinct critical temperatures and pressures. For example, methanol is supercritical at or above a temperature of approximately 240°C and pressure of approximately 81 bar (1173 psig), or equivalents. Ethanol is supercritical at or above a temperature of approximately 241°C and pressure of approximately 61 bar (890 psig), or equivalents.

The following description of exemplary embodiments, methods, processes and examples provided herein includes methanol as an exemplary alcohol used in exemplary reactions. The corresponding reaction products generated will be those produce by reactions with methanol, and those of ordinary skill in the art will understand and appreciate that other alcohols may be used in alternative embodiments, and noting that process and method embodiments as provided herein are not limited by the use of a methanol. In alternative embodiments, e.g., situations in which it is advantageous to use other alcohols, processes and provided herein includes the adjustment of reaction conditions to achieve the desired results using the alternative alcohol. In alternative embodiments, the alcohol used in the reaction contains between 1 and 5 carbons, or 1, 2, 3, 4, 5 or 6 or more carbons, e.g. methanol, ethanol, propanol, butanol, isobutanol, isopropyl alcohol or a combination thereof. In various other embodiments, conditions may be such that a higher alcohol containing more than 5 carbons are used. While in some exemplary embodiments methanol is used as the alcohol, in other embodiments other alcohols are used, and those skilled in the art would understand that other alcohols could be used.

In alternative embodiments, "biodiesel" refers to a fuel product comprised primarily or substantially of fatty acid alkyl esters derived from the transesterification of a natural oil feedstock with an alcohol. The composition of the fatty acid alkyl esters will depend on the alcohol used in the transesterification reaction. For example, if methanol is the alcohol used in the reaction, the fatty acid alkyl esters will be fatty acid methyl esters (FAME). If ethanol is the alcohol used in the reaction, the fatty acid alkyl esters will be fatty acid ethyl esters (FAEE). Various specification and standards have been established to characterize biodiesel fuels and blend stocks for example, the American Society of Testing and Manufacturing (ASTM) D6751-14 "Standard Specification for Biodiesel Fuel Blend Stock (B100) for Middle Distillate Fuels" which is incorporated herein in its entirety. In alternative embodiments, the biodiesel produced meets or exceeds those specifications established by ASTM D6751-14.

"USP-Grade glycerol" or "food-grade glycerol" is a glycerol product meeting or exceeding the standards set forth by the U.S. Pharmacopeial Convention (USP) for classification as a "USP-grade" glycerol. In alternative embodiments, the systems and methods as provided herein result in the production of USP-grade glycerol as a co-product to the production of biodiesel.

In alternative embodiments, a "co-solvent" is a product that increases the solvolysis activity of the reaction mixture, thereby enabling a more complete conversion of lipids to biodiesel and increasing the overall yields of the process. In alternative embodiments, the disclosed processes comprise the addition of a co-solvent to the reaction mixture (the mixture of the alcohol and the feedstock). The co-solvent can be, for example, a hydrocarbon or hydrocarbon mixture, or carbon dioxide (CO₂). In the disclosure that follows, CO₂ is the co-solvent used, although those skilled in the art will appreciate that other co-solvents may be substituted in alternative embodiments.

Exemplary embodiments, methods, processes and examples provided herein comprise use of a palm oil fatty acid distillate (PFAD) as the feedstock. The composition of palm oil fatty acid distillates vary depending on various factors including the specific process conditions used during their production as well the composition of the palm oil from which they were generated. An exemplary PFAD is comprised of more than 70% free fatty acids (FFAs), e.g. 80% FFAs and up to 90% or more FFAs. Other components can include glycerides (primarily triglycerides, with smaller amounts of di- and mono-glycerides), e.g. about between 10% to 25% glycerides and unsaponifiable material including vitamin E (primarily tocotrienols), e.g. between about 0.1% to 2% vitamin E, sterols, e.g. between about 0.1 to 2% sterols, and squalenes, and other materials e.g. water.

### Exemplary Process

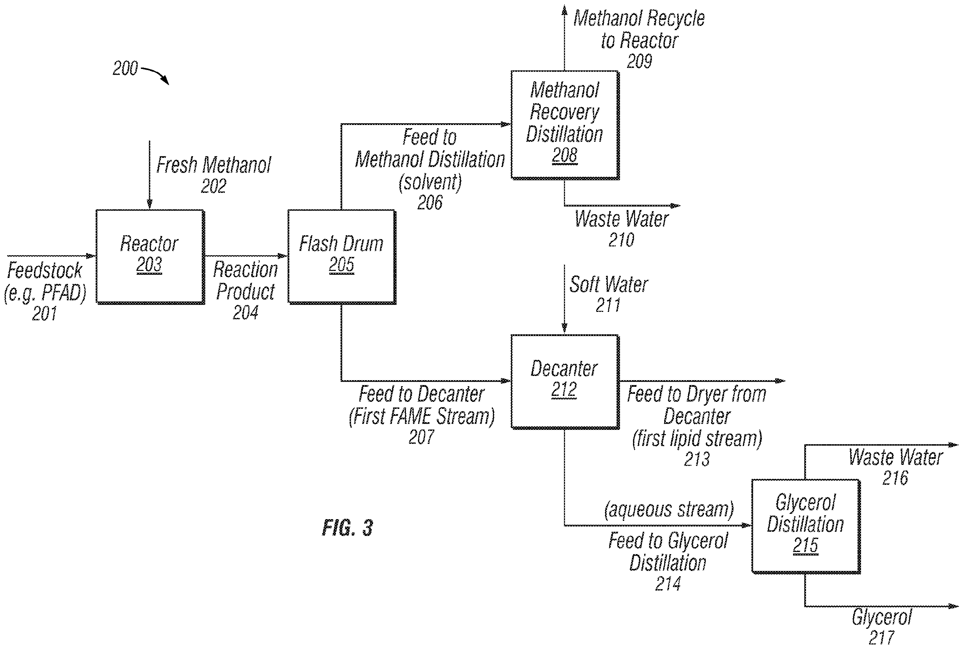

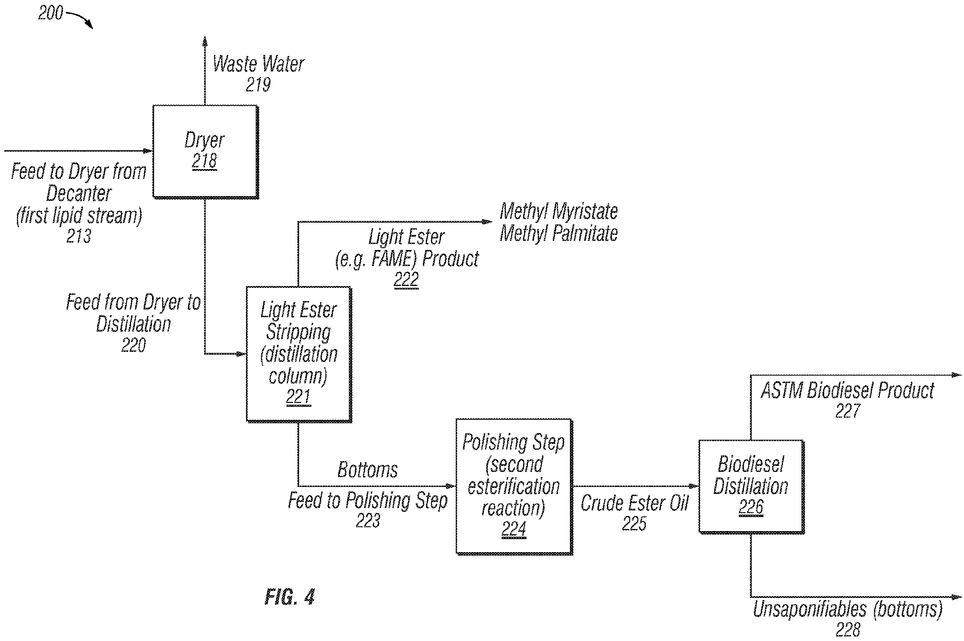

Fig. 3 and Fig. 4 illustrate an exemplary process **200** for converting PDAD to biodiesel and other products. In alternative embodiments, a PFAD feedstock comprising **201** FFAs in the amount of approximately 70% by weight of the feedstock is combined with methanol **202** (or another alcohol) in a molar ratio of alcohol-to-saponifiable-material-in-the-feedstock of between about 3:1 to 100:1, e.g. about 40:1, to form a reaction mixture. In alternative embodiments the alcohol, e.g., methanol, is essentially free of any contaminates, e.g. about 99.8% methanol, to form a reaction mixture. Once the alcohol and feedstock are combined, they are subjected to mixing, e.g. using mechanical sheer and or sonication mixing, to form an emulsion. The feedstock and alcohol can be mixed for between about 5 to about 180 minutes, e.g. about 60 minutes or an emulsion is formed. If sonication is selected as the method of mixing, the frequency range can be between about 20-100 kHz, e.g. about 42 kHz.

In alternative embodiments, the emulsified reaction mixture comprising the methanol/feedstock emulsification is then pumped or otherwise transferred into a suitable reaction vessel **203** capable of maintaining the reaction mixture at a temperature and at a pressure above the critical temperature and pressure of methanol (i.e. a temperature above about 240 °C and a pressure above about 81 bar (1173 psig)) for the desired reaction time. In alternative embodiments, the reaction vessel is a plug-flow reactor, a continuously stirred tank reactor (CSTR), or other suitable reactor. In alternative embodiments, the reactor is a plug-flow type reactor comprising a series of concentric metal heat exchangers wherein the emulsified reaction mixture is pumped into the reactor via a positive displacement pump (or other suitable pump type) comprising a backpressure regulator valve and a discharge mechanism operationally connected to the reaction vessel. In such embodiments, the hydraulic force generated by compacting the fluid reaction mixture against a back pressure regulator valve generates pressures of between about 34 bar (500 psig) to about 345 bar (5000 psig), e.g. about 138 bar (2000 psig), as measured at the discharge of the pump. In alternative embodiments, the pressurized reaction mixture passes through the discharge mechanism and into the reaction vessel wherein the pressure generated by the pump is maintained for the duration of the reaction.

In alternative embodiments, a co-solvent can be added to the methanol/feedstock reaction mixture. The co-solvent can be mixed at the same time that the methanol and feedstock are mixed, or added to the pressurized methanol/feedstock mixture in the reaction vessel via a port, for example a port following the discharge mechanism of the pump. The co-solvent can be, for example, an organic acid, e.g. carbonic acid, a hydrocarbon, e.g. methane, ethane, propane, butane, or pentane, or any combination thereof. The amount of the co-solvent in the reaction mixture (along with the methanol and feedstock), can be in the amount of between about 0.01:1 to 10:1 co-solvent-to-methanol, e.g. between about 0.05:1 to about 8:1, about 0.1:1 to about 6:1, about 0.15:1 to about 4:1, or about 0.2:1 to about 2:1, or about 0.2:1 co-solvent-to-methanol.

In alternative embodiments, the pressurized esterification/transesterification reaction mixture, comprising the feedstock, alcohol (e.g., methanol), and, if present, the optional co-solvent are then heated in a suitable reaction vessel to a temperature in the range of between about 150°C to about 450°C, e.g. 285°C, or a temperature about the critical temperature of the selected alcohol. In an exemplary embodiment, the alcohol in the esterification reaction mixture is methanol and the temperature of the reaction is above the critical temperature of methanol i.e. above about 240°C, e.g. about 285°C, and the pressure is above the critical pressure of the methanol, i.e. above about 81 bar (1174 psig) e.g. 138 bar (2000 psig).

In alternative embodiments, the esterification reaction mixture is maintained at the desired temperature and pressure and allowed to react for between about 1 minute to about 300 minutes, e.g. between about 5 minutes about 60 minutes, about 10 minutes and about 40 minutes, or about 15 minutes about 35 minutes, or about 30 minutes. During the reaction, the alcohol, e.g., methanol, reacts with (esterifies) the FFAs in the feedstock to generate fatty acid methyl esters (FAME). The methanol reacts with (transesterifies) the esters (e.g. glycerides) in the feedstock to generate FAME. In alternative embodiments, the approximately 95% of the FFAs present in the feedstock undergo an esterification reaction with the methanol to generate FAME and approximately 95% of the esters in the feedstock, e.g. glycerides, phospholipids or other esters, undergo a transesterification reaction with the methanol to generate FAME.

In alternative embodiments, the product mixture **204** generated by the esterification/transesterification reaction, referred to herein as the "first product mixture," **204** can comprise FAME (or equivalent alkyl esters if an alcohol other than methanol is used), unreacted alcohol, unreacted FFAs, unreacted esters e.g. glycerides, glycerol, co-solvent (if present in the reaction mixture) and possibly other products, e.g. glycerol phosphatidyls if phospholipids are present in the feedstock, vitamin E compounds, and sterols or any combination thereof. The portion of unreacted esters after the esterification reaction can be between about 0.1% to about 20% of the esters that were present in the esterification feedstock, e.g. between about 1 and 15%, about 2 and 10% or about 5% of the esters that were present in the esterification feedstock. The portion of unreacted free fatty acids after the esterification reaction can be between about 0.1% to about 20% of the free fatty acids that were present in the esterification feedstock, e.g. between about 1 and 15%, about 2 and 10% or about 5% of the free fatty acids that were present in the esterification feedstock

In alternative embodiments, following the reaction, the first product mixture (i.e. the product mixture in which the fatty acids generated in the first stage of the process are substantially esterified and the esters are substantially transesterified) is discharged from the reactor via a high-pressure pump and passed through a high pressure concentric heat exchanger (wherein the pressure is maintained by the backpressure regulator) wherein the heat is withdrawn from the product mixture and optionally recovered (where the heat can be recycled for use elsewhere in the process, e.g. to heat the reactor, thereby decreasing the overall energy requirements of the system. The mixture then passes through a backpressure regulator device at a temperature of between about 125°C to about 350°C, or between about 150°C to about 300°C, e.g. about 215°C.

In alternative embodiments, following the heat recovery step, the first product mixture is optionally subjected to a flash separation process wherein the pressure of the cooled esterification product mixture is reduced by, for example, passing the product mixture through a backpressure regulator device and into a flash drum **205** or other appropriate or equivalent vessel wherein the pressure of the first product mixture is reduced from the pressure within the heat exchanger (e.g. above about 81 bar (1171 psig) or about 138 bar (2000 psig)) to about atmospheric pressure (i.e. about 1 bar (14.7 psig)). In alternative embodiments, the pressure of the first product mixture is decreased rapidly and the decrease in pressure in an environment in which the vapor pressure of the alcohol exceeds its external pressure (the pressure of the flash drum or vessel), allowing for the alcohol, co-solvent (if present) and any water (collectively referred to as "the solvent" in this and subsequent steps) **206** to vaporize or "flash" out of the product mixture.

In alternative embodiments, the optional flash step causes approximately 95% of the solvent **206** present in the product mixture to vaporize and leave the flash vessel, with approximately 5% of the solvent remaining in a liquid state and exiting the bottom of the flash unit along with the remaining products in the product mixture, referred to herein as the "first FAME stream" (or equivalent alkyl esters if an alcohol other than methanol is used) **207.** In such embodiments, the concentration of solvent (i.e. alcohol/ and optionally water and/or the co-solvent) leaving the flash unit in a liquid state (in the first FAME stream) is approximately 2 wt.% of the first FAME stream.

In alternative embodiments, the first FAME stream (comprising FAME and glycerol, any unreacted FFAs and/or esters e.g. glycerides, as well as the solvent that was not separated in the previous flash step) leaves the flash unit at a temperature in the range of between about 110 to about 125 °C, e.g. 115 °C and is optionally sent to a heat exchanger, e.g. a standard shell and tube heat exchanger, wherein it is cooled to about 95 °C. The recovered heat can be recycled for use in the process, e.g. to heat the reactor.

In alternative embodiments, the solvent mixture **206,** wherein the mixture is approximately 90 wt % methanol or 90 wt % methanol/co-solvent (if co-solvent is present) and approximately 10 wt % water is then distilled to yield a substantially pure methanol product **209,** e.g. approximately 99.8% or more methanol. The distillation unit **208** can comprise, for example, a packed or trayed distillation columns, e.g. a trayed distillation column comprising between 1 and 75 stages, e.g. between 5 and 70 stages, between 10 and 65 stages, between 15 and 60 stages, between 20 and 55 stages, between 25 and 50 stages, or between 30 and 45 stages, e.g. 25 stages. The distillation is achieved under a vacuum of between about 0.34 bar (5 psig) and 1.4 bar (20 psig) e.g. 1 bar (14.7 psig) to generate a substantially pure methanol product **209.** The generated substantially pure methanol product **209** can be recycled to the alcohol supply tank for use in subsequent reactions. If present the co-solvent is distilled in the same distillation step to yield a substantially pure co-solvent product, e.g. 99.8% co-solvent. The substantially pure co-solvent can be recycled to for use in subsequent reactions. The bottoms of the methanol recovery distillation column are a wastewater product **210.**

In alternative embodiments, after the first FAME stream is optionally cooled via a heat exchanger, it is transferred to mixing vessel wherein it is mixed with water via, for example, an inline static mixer or wherein it is mixed with soft water in a ratio of about 50:1 first FAME stream-to-water by mass, or in a ratio of 1:1 water-to-glycerol by mass. In alternative embodiments, the mixture of the water and the first FAME stream mixture is then transferred to a suitable separation vessel **212,** e.g. a decanter, a centrifuge, or a hydrocyclone or series of hydrocyclones, wherein a lipid stream, referred to herein as the "first lipid stream" **213** and an aqueous stream **214** are formed and are separated.

In alternative embodiments, the aqueous stream **214** that leaves the decanter comprises alcohol, water (including any water that was not removed in the flash separation step and water introduced in the present glycerol recovery/water-wash step) and glycerol, is then transferred to a glycerol stripping column **215,** e.g. a 6-stage stripping column, in which the aqueous stream **214** is introduced to the top of the column **215** and, upon contacting the bottom of the column is heated such that a vapor phase **216,** comprising primarily alcohol and water, is generated and rises to the top of the column where it is removed. In this exemplary embodiment, the column "bottoms" are a primarily a glycerol product **217** in the range about 85 to about 99.9 wt % glycerol, e.g. about 99.5% glycerol, which can be marketed directly as "splitter crude" grade glycerol or upgraded through techniques known in the art to a USP grade tech glycerol.

In alternative embodiments, the first lipid stream **213,** having been isolated in the decanter **212** or other suitable device or vessel, is then heated to between about 150 °C to about 220 °C via a shell-and-tube heat exchanger **218** and is allowed to flash at an absolute pressure in the range of between about 0 bar (0 psig) to about 0.7 bar (10 psig), e.g. 0.07 bar (1 psig). In this flash step, any excess water contained **219** in the lipid stream from the decanting step is removed, thereby "drying" the first lipid stream in order to meet the water content specifications for ASTM B 100 biodiesel.

In alternative embodiments, the "bottoms" **220** of this flash/drying unit are then sent to a distillation column **221** wherein the FAME is separated from the other products present in the lipid stream, e.g. waxes, unreacted esters e.g. glycerides, unreacted FFAs, vitamin E (tocopherols/tocotrienols), sterols, or the like to yield a distillate stream **222** comprising substantially pure e.g. 98.5% or more, FAME. In alternative embodiments, the distillation column **221** can be, for example a packed distillation column or a trayed distillation column. In alternative embodiments, the distillation column **221** comprises between 1 and 50 stages, e.g. between 5 and 45 stages, between 10 and 40 stages, between 15 and 35 stages, between 20 and 30 stages, or 25 stages. In alternative embodiments, the distillation is conducted under a vacuum in the range of between about 0.001 and 0.27 bar (about 1 and 200 Torr absolute), e.g. between about 0.003 and 0.2 bar (about 2 and 150 Torr), between 0.005 and 0.133 bar (4 and 100 Torr), between 0.008 and 0.07 bar (6 and 50 Torr), between 0.01 and 0.03 bar (8 and 20 torr), or about 0.013 bar (about 10 Torr absolute). In alternative embodiments wherein methanol is the alcohol used in the esterification reaction, the distillate stream **222** comprises substantially pure FAME (or equivalent alkyl esters if an alcohol other than methanol is used) meeting or exceeding the standards established for ASTM B100-grade biodiesel.

In alternative embodiments, the distillation column **221** is configured such that the vapor (distillate) stream **222** generated in the distillation column **221** comprising the FAME will be comprised primarily of methyl palmitate and other light FAME molecules, i.e. those FAME molecules with a vapor pressure higher than that of palmitic acid. In alternative embodiments, the vapor stream **222** comprising light FAME molecules (FAME molecules with 16 or fewer carbons) can be further processed to generate product streams of individual FAMEs, e.g. a product stream of purified methyl palmitate.

In alternative embodiments, the bottoms stream **223** of the distillation column comprises those products in the first lipid stream with vapor pressures lower than methyl palmitate including, for example, "heavy" FAMEs (those FAMEs with more than 16 carbons), unreacted FFAs, unreacted esters e.g. glycerides, and any unsaponifiable material. In alternative embodiments, the bottoms stream **223** can be further processed to separate discreet, high-value product streams e.g. a substantially pure vitamin E product, a substantially pure sterol product, a substantially pure squalene product, or other product streams.

In alternative embodiments, either before or after discreet product streams have been isolated from the bottoms stream **223** comprising the heavy unreacted, saturated FFAs, the remaining products are subjected to "polishing step" **224** i.e. a second esterification/ transesterification reaction, either acid-catalyzed (e.g. using a strong cation exchange resin packed in a pipe) or non-catalytic, to convert the majority, i.e. 95% or more, of the unreacted FFAs and unreacted esters, e.g. glycerides, from the first esterification/transesterification reaction, to FAME or other equivalent alkyl ester if an alcohol other than methanol is used in the reaction. By subjecting the bottoms stream **223** generated from the distillation described above to a second esterification reaction, the problem of heavy FAME molecules not being easily separated from unsaturated FFAs with similar vapor pressures (primarily C18 FAMEs and palmitic acid, if PFAD is the feedstock) is overcome. In alternative embodiments, the second esterification/transesterification reaction **224** serves to convert approximately 95% of the unreacted FFAs and esters from the first esterification/transesterification reaction into FAME (or equivalent alkyl esters if an alcohol other than methanol is used), which, after undergoing a second distillation as described above, generates a substantially purified FAME product that can be blended with the purified FAME product from the first reaction to. In this way, the overall biodiesel yields of the process justify the use of feedstocks comprising high FFA content, wherein a large portion of the fatty acid profile, e.g., about 40% to 55% of the fatty acids in the feedstock, are saturated fatty acids.

In alternative embodiments, the second esterification/transesterification reaction proceeds identically to the first esterification/transesterification reaction but wherein the feedstock for the reaction is the "bottoms" product generated from the distillation of the lipid stream generated in the first esterification/transesterification reaction. In alternative embodiments, following the second esterification/ transesterification reaction, the generated reaction product **225** is subjected to the same processing steps described above following the first esterification/transesterification reaction to generate a substantially purified FAME product that meets or exceeding the standards established for ASTM B 100-grade biodiesel. In alternative embodiments, the reaction product of the second esterification reaction **225** subjected to a distillation step **226** (as described above) to generate an ASTM Biodiesel product **227** which can be blending with the ester stream **222** generated from the first esterification reaction. The bottoms of the distillation column **228** comprise the unsaponifiable material contained in the starting feedstock.

### Process for the conversion of low-FFA oils with a high percentage of saturated fatty acids

In alternative embodiments, the feedstock in the process is an oil or lipidcomprising product comprising a high percentage (e.g. between about 35-60%) of saturated fatty acids with a relatively low percentage (e.g. less than about 10%) of free fatty acids, e.g. crude palm oil. In embodiments where such a feedstock is used, the process comprises a first hydrolysis reaction to hydrolyze the glycerides in the feedstock to generate glycerol and FFAs, and an esterification/transesterification reaction wherein the resulting generated FFAs from the hydrolysis stage are reacted with an alcohol at a temperature above the critical temperature of the alcohol and a pressure above the critical pressure of the alcohol to generated fatty acid alkyl esters.

The product generated in the esterification/transesterification reaction is separated into a "light" fraction comprising the lighter alkyl esters (i.e. alkyl esters with 16 or fewer carbons) and a "heavy" fraction comprising heavy alkyl esters (e.g. alkyl esters with more than 16 carbons) and any unreacted FFAs.

In alternative embodiments, the esterification/transesterification reaction converts approximately 95% of the FFAs and glycerides to fatty acid alkyl esters. In order to generate a biodiesel product meeting the specification set forth in relevant industrial standards, e.g. ASTM Specification D6751-14, generated product must be distilled to or otherwise purified to increase the percentage of alkyl esters in the final product. In alternative embodiments, the feedstock comprises high percentage of saturated fatty acids, e.g. 40% or more saturated fatty acids (for example, a crude palm oil). Unreacted saturated free fatty acids, e.g. palmitic acid, in the esterification/transesterification product have very similar vapor pressures to the lighter alkyl esters, e.g. methyl stearate, making the isolation of a pure (98% or more) alkyl ester product difficult. Alternative exemplary embodiments overcome this problem by separating the lighter alkyl esters generated in the esterification/ transesterification reaction from the "bottoms" comprising the heavier (i.e. longer carbon chains) alkyl esters and unreacted FFAs, and any esters (e.g. glycerides and phospholipids) or other saponifiable material. The bottoms are then subjected to a second reaction or processing step, e.g. a second esterification/transesterification reaction, wherein the majority (e.g. 95% or more) of the unreacted FFAs and esters from the first esterification/transesterification product are converted to alkyl esters. The resulting product is suitably purified to meet the relevant industrial standards for biodiesel and can optionally be combined with the separated lighter alkyl esters separated from the initial esterification/transesterification reaction.

### Exemplary Process

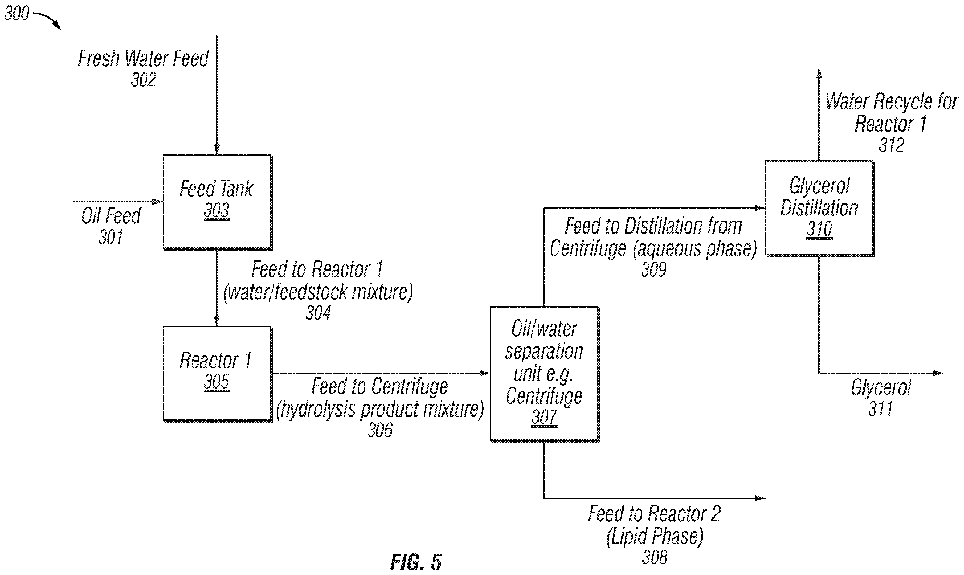

Figs. 5-7 are a schematic representation of a process **300** for converting a natural oil feedstock with a high percentage of saturated fatty acids to biodiesel. In alternative embodiments, the system is comprised of a first hydrolysis unit and a second esterification unit. In alternative embodiments, the feedstock undergoes a first hydrolysis reaction, e.g. a non-catalytic hot compressed water hydrolysis reaction to hydrolyze ester bonds in the feedstock to generate FFAs from glycerides and other lipids, e.g. phospholipids. The product mixture resulting from the first hydrolysis stage of the process comprising the FFAs is sent to an esterification unit where is reacted with an alcohol at a temperature and pressure above the critical temperature and pressure of the alcohol to generate fatty acid alkyl esters.

### Hydrolysis Reaction (Stage 1)

In alternative embodiments, the natural oil feedstock **301** comprising primarily esters (e.g. triglycerides) and having a high percentage of saturated fatty acids, e.g. crude palm oil (CPO), is combined, in a feed **302** tank with water **303,** e.g. deionized water, in a molar ratio of water-to-feedstock of between about 3:1 to about 100:1, e.g. between about 10:1 to about 80:1, 20:1 to about 60:1, or about 40:1 water-to-feedstock. Following the combination of water **302** and the feedstock **301,** the water/feedstock mixture **304** can optionally be mixed mechanically to form an emulsion using, e.g. mechanical sheer and/or ultrasonication. The water/feedstock mixture (having optionally been emulsified) is then pumped or otherwise transferred to a reaction vessel **305** e.g. a Plug Flow, Continuously Stirred Tank, or other suitable reaction vessel using, for example, a positive displacement pump. In alternative embodiments, the water/feedstock mixture is pumped into the reaction vessel pressurized to between about 34 bar (500 psig) to 345 bar (5000 psig), e.g. 69 bar (1000 psig) to 207 bar (3000 psig), or about 138 bar (2000 psig), wherein the pressure in the reaction vessel is created hydraulically by compacting the fluid water/feedstock mixture against a back pressure regulator valve calibrated to maintain a desired pressure in the reaction vessel for the duration of the reaction.

In alternative embodiments, a co-solvent can optionally be added to the water/feedstock reaction mixture. If a co-solvent is added, it is added directly after the discharge of the high-pressure pump via a port where the co-solvent is added to the already pressurized reaction mixture. The co-solvent can be, for example, an organic acid or a hydrocarbon, or a combination thereof. If added to the reaction mixture, the amount of co-solvent added to the reaction mixture is in the co-solvent-to-water ratio of between about 0.01:1 to 10:1 e.g. between about 0.2:1 co-solvent-to-water.

In alternative embodiments, the contents of the reaction vessel are allowed to react at the selected temperature and pressure for a period of between about 1 to about 300 minutes, e.g. about 2 to about 250 minutes, about 4 to about 200 minutes, about 6 to about 150 minutes, about 8 to about 100 minutes, about 10 to about 90 minutes, about 12 to about 70 minutes, about 14 to about 50 minutes, about 16 to about 40 minutes, about 18 minutes to about 30 minutes, or about 20 minutes, or until substantially all, or most ( 70% or more of the ester bonds, e.g. 75%, 80%, 90%, 95%, 97%, 98%, 99% or more) of the ester bonds in the feedstock have been hydrolyzed, thereby "cleaving" or separating, via hydrolysis acting at the ester bonds of the esters in the feedstock, fatty acid molecules to generate "free" (un-esterified) fatty acids.

In alternative embodiments, after the hydrolysis reaction mixture (feedstock, water, and optionally a co-solvent) **306** has been reacted for the desired period of time, the resulting "hydrolysis product mixture" **306** will vary depending on the composition of the feedstock, but may comprise, for example, free fatty acids, glycerol, water, unsaponifiable material (e.g. waxes, sterols and hydrocarbons if present in the feedstock), and glycerol phosphatidyls (resulting from the cleaving of the free fatty acids from phospholipids if phospholipids are present in the feedstock), as well as any unreacted (un-hydrolyzed) esters e.g. glycerides, and phospholipids.

In alternative embodiments, a heat-recovery unit operation is included in the process wherein, following the hydrolysis reaction, incoming hydrolysis reaction mixture material (feedstock, water and optionally a co-solvent) **306** is heated with the heat contained in the hydrolysis product mixture using heat-exchanger device, e.g. a shell-and-tube heat exchanger or other suitable heat recovery system. In alternative embodiments, a shell-and-tube heat exchanger is utilized and comprises an outer cylindrical tube or "shell" having an exterior wall and an interior wall defining an internal cavity within which one or more tubes are contained, each having a smaller diameter than the outer tube, and each having an exterior wall and an interior wall defining an internal cavity.

In an exemplary embodiment, a shell-and-tube heat exchanger is utilized in the process and the heated material (the hydrolysis product mixture), flows within the "tube" portion (within the interior cavity of the tubes contained within the shell) of the shell-and-tube heat exchanger and the incoming process material, having just exited the discharge of the high-pressure pump and therefore pressurized to the desired pressure of the hydrolysis reaction, flows counter-currently within the "shell" of the shell-and-tube heat exchanger, (between the exterior walls of the tubes contained within the shell and the interior wall of the shell). Heat is thereby transferred and simultaneously heats the incoming reaction mixture and cools the hydrolysis product mixture **306.**

The temperature of the hydrolysis product mixture **306** can be decreased from the temperature of the hydrolysis reaction by, for example, between about 70 °C and about 370 °C, depending on the temperature of the hydrolysis reaction and the desired temperature of the product mixture in subsequent unit operations. In certain embodiments, the temperature of the reaction vessel is maintained at a temperature of 300 °C during the hydrolysis reaction and the hydrolysis product mixture **306** is cooled to a temperature of 95 °C in the foregoing eat exchange step, a reduction in temperature of 205 °C. In other embodiments, the hydrolysis reaction mixture **306** is conducted at higher or lower temperatures and the hydrolysis reaction products are cooled to higher or lower temperatures than 95 °C in the heat exchange step.

In alternative embodiments, following the heat-exchange, the pressure of the cooled hydrolysis product mixture **306** is reduced by, for example, passing the reaction products through a backpressure regulator device that decreases the pressure of the product mixture to about atmospheric pressure (i.e. 1 bar (14.7 psigg)). In alternative embodiments, the pressure of the hydrolysis reaction products is decreased rapidly and a portion of the water in the product mixture "flashes" off, i.e. vaporizes, as the pressure exerted on the reaction products is reduced to below the vapor pressure of the cooled mixture. Any suitable vessel known in the art may be used for this step and is therefore not limited by a specific apparatus or device. The flashed water can be captured and recycled in the process for subsequent hydrolysis reactions.

In alternative embodiments, the product mixture **306** is then transferred to an "oil/water separation unit" **307** e.g. a centrifuge, decanter, hydrocyclone (or series of hydrocyclones), or other suitable apparatus or system wherein the product mixture is separated into a lipid phase **308** and an aqueous phase **309,** and the lipid **308** and aqueous phases **309** are physically separated from one another thereby generating two separate streams for further processing. In alternative embodiments, the lipid phase **308** comprises the free fatty acids and possibly other lipids (if all of the ester bonds in the feedstock was not completely hydrolyzed) e.g. glycerides and phospholipids, and an aqueous phase **309** comprising water and glycerol and, if phospholipids were present in the feedstock, glycerol phosphatidyls. In alternative embodiments, the lipid phase **308** floats on top of the aqueous phase **309** due to the differences in density of the products within each phase and the lipid phase **308** is removed from the aqueous phase.

In alternative embodiments, the separated lipid phase **308** is subjected to an optional "drying" step wherein any water that was entrained in the lipid during the lipid phase **308** separation step is removed from the remaining lipid products (e.g. free fatty acids and glycerides), thereby generating a lipid product substantially free of water. In alternative embodiments, the drying is achieved by heating the lipid phase **308** to a temperature of between about 40 °C and about 220 °C, e.g. between about 100 °C and about 195 °C, about 120 °C and about 190 °C, about 140 °C and about 185 °C, or about 185 °C under a vacuum of between about 0.007 to about 1 bar (about 5 to about 770 Torr absolute), e.g. between about 0.013 and about 0.8 bar (about 10 and about 600 Torr absolute), between about 0.02 to about 0.67 bar (about 15 and about 500 Torr absolute), between about 0.27 and about 0.53 bar (about 20 and about 400 Torr absolute), between about 0.04 and about 0.4 bar (about 30 and about 300 Torr absolute), between about 0.047 and about 0.27 bar (about 35 and 200 Torr absolute), between about 0.05 and about 0.13 bar (about 40 and about 100 Torr absolute), between about 0.06 and about 0.1 bar (about 45 and about 80 Torr absolute), between about 0.07 and about 0.08 bar (about 50 and about 60 Torr absolute), or about 0.073 bar (about 55 Torr absolute). The water that has been removed from the lipid phase can optionally be recycled in the process.

In alternative embodiments, the aqueous phase **309** generated in the lipid separation step **307** is transferred to a distillation column, stripping column, or other suitable separation column or device **310,** wherein the glycerol **311** is separated from the remaining products in the aqueous phase. The configuration of the column (e.g. the stripping column or distillation column) **310** can vary depending on the desired product output and composition of the aqueous phase **309** that is the input stream to the column. In alternative embodiments, the distillation column **310** is a packed distillation column. In other embodiments, the distillation column **310** is a trayed distillation column comprising between 1 and 50 stages, e.g. between 2 and 40 stages, between 3 and 30 stages, between 4 and 20 stages, between 5 and 10 stages, or 6 stages. In alternative embodiments, the aqueous phase **310** is transferred to a glycerol distillation column, e.g. a 6-stage distillation column, in which the aqueous stream **309** is introduced into the column and is heated such that a vapor phase, comprising primarily water, or water and alcohol (if the input to the glycerol distillation unit includes the glycerol-containing aqueous phase generated in the second stage of the process) **312,** is generated and rises to the top of the column where it is removed. In this exemplary embodiment, the column "bottoms" are a primarily a glycerol product **311** in the range about which can be marketed directly as "splitter crude" grade glycerol or upgraded through techniques known in the art to a USP grade tech glycerol. In alternative embodiments, the aqueous phase **309** is distilled under a vacuum of between about 0.013 and 1 bar (about 10 and 770 Torr absolute), e.g. between about 0.067 and about 0.67 bar (about 50 and about 500 Torr absolute), about 0.13 bar and about 0.53 bar (about 100 and about 400 Torr absolute), about 0.27 and about 0.4 bar (about 200 and about 300 Torr absolute), or about 0.33 bar (about 250 Torr absolute). The distillate stream generated in the distillation column is deionized water **312,** which can be recycled in in the process for use in subsequent hydrolysis reactions.

### Esterification Reaction (Stage 2)

In alternative embodiments, the lipid phase **308** generated in the foregoing lipid separation step following the hydrolysis reaction and comprising free fatty acids (FFAs), and possibly esters e.g. glycerides and/or phospholipids referred to herein as the "esterification feedstock" **308** is combined with an alcohol **313,** e.g. methanol or ethanol, that is essentially free of any contaminants, e.g. about 99.0% alcohol, to form a reaction mixture. In certain embodiments, an alcohol with lower purity may be used, e.g. an alcohol comprising about 95% alcohol and 5% water. Lower-purity alcohols are generally cheaper than high-purity alcohols and there use may therefore result in more favorable economics despite lower FFA yields from the process. The lipid phase generated in the first stage of the process **308** is therefore the feedstock for the second stage of the process. The molar ratio of the alcohol to the esterification feedstock **308** in the reaction mixture can be between about 5:1 to about 70:1, e.g. about 40:1. In alternative embodiments, the moisture content (amount of water) of the esterification feedstock **308,** is between about 0 and 5% by weight of the feedstock **308.** Once the esterification feedstock **308,** and alcohol **313** are combined, they are optionally mixed, e.g. via mechanical sheer and or sonication mixing, to form an emulsion or equivalent. The esterification feedstock and alcohol can be mixed for between about 5 to about 180 minutes, e.g. about 60 minutes or an emulsion is formed. If sonication is selected as the method of mixing, the frequency range can be between about 20-100 kHz, e.g. about 42 kHz. The combined and optionally emulsified esterification feedstock and alcohol mixture is referred to herein as the "esterification reaction mixture."

In alternative embodiments, the esterification reaction mixture is then pumped into a reactor **314** comprising a series of heat exchangers, e.g., concentric metal heat exchangers, via a positive displacement pump (or other suitable pump type) wherein the pressure created from pumping the mixture against a backpressure regulator valve on the reaction mixture is between about 34 bar (500 psig) to about 345 bar (5000 psig), e.g. about 138 bar (2000 psig), as measured at the discharge of the pump. Directly after the discharge of the high-pressure pump, a co-solvent, e.g. an organic acid or a hydrocarbon e.g. methane, ethane, propane, butane, or pentane or any combination thereof, may optionally be added to the esterification reaction mixture via a port that is operationally connected to the discharge area of the pump. The amount of optional co-solvent-to-alcohol in the esterification reaction mixture can be, for example a molar ratio of between about 0.01:1 to about 5:1, e.g. about e.g. between about 0.05:1 to about 8:1, about 0.1:1 to about 6:1, about 0.15:1 to about 4:1, or about 0.2:1 to about 2:1, or about 0.2:1 co-solvent-to-alcohol.

In alternative embodiments, the pressurized esterification reaction mixture, comprising the esterification feedstock, alcohol, and the optional co-solvent and/or FFAs are then heated in a suitable reaction vessel to a temperature in the range of between about 200°C to about 400°C, e.g. 290°C, or a temperature about the critical temperature of the selected alcohol. In an exemplary embodiment, the alcohol in the esterification reaction mixture is methanol and the temperature of the reaction is above the critical temperature of methanol, i.e., above about 240°C, e.g. about 300°C, and the pressure is above the critical pressure of the methanol, i.e. about 81 bar (1174 psig). The esterification reaction mixture is maintained at the desired temperature and pressure and allowed to react for between about 1 minute to about 300 minutes, e.g. between about 5 minutes about 60 minutes, about 10 minutes and about 40 minutes, or about 15 minutes about 25 minutes, or about 20 minutes. During the reaction, the alcohol esterifies the free fatty acids to generate fatty acid alkyl esters, e.g. fatty acid methyl esters (FAME) if methanol is the alcohol used in the reaction. The alcohol undergoes a transesterification reaction with the esters (if present) in the reaction mixture to generate fatty acid alkyl esters. In alternative embodiments, substantially all of the FFAs present in the feedstock undergo an esterification reaction with the alcohol to generate fatty acid alkyl esters and substantially all of the esters in the feedstock, e.g. glycerides, phospholipids or other esters, will similarly be subjected to transesterification to generate fatty acid alkyl esters.

If water is present in the esterification reaction mixture, the water can allow for less severe reaction conditions, e.g. lower temperatures and pressures, by increasing the solvolysis activity of the mixture, relative to a mixture comprising alcohol and the esterification feedstock alone i.e. without water. The water can also react with a portion of the ester bonds present in the esterification feedstock, thereby hydrolyzing a portion of the esters to generate free fatty acids. The hydrolysis of esters by water can allow for increased free fatty acid yield from the esterification reaction with decreased reaction times. In alternative embodiments, during the second stage of the process, the esterification reaction allows for the simultaneous hydrolysis and esterification of esters in the esterification feedstock. As an example, a triglyceride in the esterification feedstock may be subjected to hydrolysis with water to generate one molecule of glycerol and 3 molecules of free fatty acids. In the same reaction step, the generated 3 free fatty acids molecules can undergo an esterification reaction with the alcohol in the esterification reaction mixture to generate three molecules of fatty acid alkyl esters.

In alternative embodiments, the product mixture generated by the esterification reaction, referred to herein as the "esterification product mixture," **315** can comprise fatty acid alkyl esters, water, unreacted alcohol, glycerol, co-solvent (if present in the reaction mixture) and possibly other products, e.g. glycerol phosphatidyls is phospholipids are present in the feedstock. In alternative embodiments the esterification product mixture **315** may also comprise esters that did not undergo a hydrolysis or transesterification reaction and therefore remain "unreacted." The portion of unreacted esters after the esterification reaction can be between about 0.1% to about 20% of the esters that were present in the esterification feedstock, e.g. between about 1 and 15%, about 2 and 10% or about 2% of the esters that were present in the esterification feedstock **308.** The esterification product mixture **315** may also comprise free fatty acids (FFAs) that did not react with the alcohol to generate fatty acid alkyl esters and therefore remain "unreacted." The portion of unreacted free fatty acids after the esterification reaction can be between about 0.1% to about 20% of the free fatty acids that were present in the esterification feedstock, e.g. between about 1 and 15%, about 2 and 10% or about 3% of the free fatty acids that were present in the esterification feedstock **308.**

In alternative embodiments, following the reaction, the esterification product mixture (i.e. the product mixture in which the fatty acids generated in the first hydrolysis stage of the process are substantially esterified and the esters that were not hydrolyzed in the first hydrolysis stage of the process are substantially transesterified) **315** is discharged from the reactor, e.g., via a high-pressure pump, and passed through a heat exchanger, e.g., a high pressure concentric heat exchanger (wherein the pressure is maintained by the backpressure regulator), and wherein the heat is withdrawn from the product mixture and optionally recovered, for example, where the heat is recycled for use elsewhere in the process, e.g. to heat the reactor, thereby decreasing the overall energy requirements of the system. In alternative embodiments, the mixture then passes through a backpressure regulator device at a temperature of between about 125°C to about 350°C, or between about 150°C to about 300°C, e.g. about 240°C.

In alternative embodiments, following the heat recovery step, the esterification product mixture **315** is optionally subjected to a flash separation process wherein the pressure of the cooled esterification product mixture is reduced by, for example, passing the product mixture through a backpressure regulator device and into a flash drum **316** or other appropriate or equivalent vessel wherein the pressure of the product mixture **315** is reduced from the pressure within the heat exchanger (e.g. above about 81 bar (1171 psig) or about 138 bar (2000 psig)) to about atmospheric pressure (i.e. about 1 bar (14.7 psig)). In alternative embodiments, the pressure of the esterification product mixture **315** is decreased rapidly and the decrease in pressure. The decrease in pressure results in an environment in which the vapor pressure of the alcohol exceeds its external pressure (the pressure of the flash drum or vessel **316**), allowing for the alcohol, co-solvent (if present) and any water (collectively referred to as "the solvent" in this and subsequent steps) **317** to vaporize or "flash" out of the product mixture.

In alternative embodiments, the optional flash step causes approximately 95% of the solvent present in the product mixture to vaporize and leave the flash vessel, with approximately 5% of the solvent remaining in a liquid state and exiting the bottom of the flash unit along with the remaining products in the product mixture, referred to herein as the "ester stream **308."** In such embodiments, the concentration of solvent **317** (i.e. alcohol/ and optionally water and/or the co-solvent) leaving the flash unit in a liquid state (in the ester stream) is approximately 2 wt.% of the ester stream **318.**

In alternative embodiments, the ester stream (comprising fatty acid alkyl esters e.g. FAME and glycerol, any unreacted free fatty acids and/or esters e.g. glycerides, as well as the water and alcohol that was not separated in the previous flash step) **318** leaves the flash unit at a temperature in the range of between about 110 to about 125°C, e.g., 115°C and is optionally sent to a heat exchanger, e.g. a standard shell and tube heat exchanger, wherein it is cooled to about 95°C. The recovered heat can be recycled for use in the process, e.g. to heat the reactor.

In alternative embodiments, the solvent mixture (the alcohol/water/ and, if present, co-solvent mixture obtained from the previous flash separation step) **317,** wherein the mixture is approximately 95 wt % alcohol or 95 wt %alcohol/co-solvent (if co-solvent is present) and approximately 5 wt % water is then distilled **319** to yield a substantially pure alcohol product, e.g., a substantially pure methanol product **320,** e.g. approximately 99.8% or more alcohol. The distillation unit **319** can comprise, for example, a packed or trayed distillation columns, e.g., a trayed distillation column comprising between 1 and 75 stages, e.g. between 5 and 70 stages, between 10 and 65 stages, between 15 and 60 stages, between 20 and 55 stages, between 25 and 50 stages, or between 30 and 45 stages, e.g. 40 stages. The distillation is achieved under a vacuum of between about 0.34 bar (5 psig) and 1.4 bar (20 psig) e.g. 1 bar (14.7 psig) to generate a substantially pure alcohol product. The generated substantially pure alcohol product **320** can be recycled to the alcohol supply tank for use in subsequent reactions. If present the co-solvent is distilled in the same distillation step to yield a substantially pure co-solvent product, e.g. 99.8% co-solvent. The substantially pure co-solvent can be recycled to for use in subsequent reactions. The "bottoms" of the alcohol distillation unit **319** are a wastewater product **321.**

In alternative embodiments, after the ester stream **318** is optionally cooled via a heat exchanger, it is transferred to mixing vessel wherein it is mixed with water **322** via, for example, an inline static mixer wherein it is mixed with soft water in a ratio of about 50:1 ester stream-to-water by mass, or in a ratio of 1 g water-to-glycerol by mass. The water **322** and ester stream **318** mixture is then transferred to a suitable separation vessel **323,** e.g. a decanter, a centrifuge, or a hydrocyclone or series of hydrocyclones, wherein a lipid stream **324,** referred to herein as the "biodiesel stream" and an aqueous stream **325** are formed and are separated.

In alternative embodiments, the aqueous stream that leaves the decanter comprises alcohol, water (including any water that was not removed in the flash separation step and water introduced in the present glycerol recovery/water-wash step) and glycerol, is then transferred to a glycerol stripping column, e.g. a 6-stage stripping column, in which the aqueous stream is introduced to the top of the column and, upon contacting the bottom of the column is heated such that a vapor phase, comprising primarily alcohol and water, is generated and rises to the top of the column where it is removed. In this exemplary embodiment, the column "bottoms" are a primarily a glycerol product in the range about 85 to about 99.9 wt % glycerol, e.g. about 99.5% glycerol, which can be marketed directly as "splitter crude" grade glycerol or upgraded through techniques known in the art to a USP grade tech glycerol. The generated glycerol product can optionally be mixed with the glycerol product generated during the first hydrolysis stage of the process. In alternative embodiments, the aqueous stream generated in the first (hydrolysis) stage of the process comprising glycerol is combined with the aqueous stream generated in the second (esterification) stage of the process and are distilled simultaneously to generate the glycerol product.

In alternative embodiments, the biodiesel stream **324** separated from the decanter **323** is then heated to between about 150 °C to about 220 °C via a shell-and-tube heat exchanger (i.e. flash separation unit) **326** and is allowed to flash at an absolute pressure in the range of between about 0 bar (about 0 psig) to about 0.7 bar (about 10 psig), e.g. 0.07 bar (1 psig), or between about 0.007 bar (about 5 torr) and 1 bar (770 torr), e.g. 0.013 bar (10 torr) to about 0.4 bar (about 300 torr), between 0.027 bar (20 torr) and 0.2 bar (150 torr), between 0.04 bar (30 torr) and 0.133 bar (100 torr), between 0.053 bar (40 torr) and 0.107 bar (80 torr), or about 0.073 bar (about 55 torr). In this flash step, substantially any excess water contained in the biodiesel stream from the decanting step is removed **327,** thereby "drying" the biodiesel fraction in order to meet the water content specifications for ASTM B100 biodiesel, if methanol is the alcohol used in the esterification reaction. In alternative embodiments, a portion of the fatty acid alkyl esters (e.g. less than about 5%) in the flash process stream is evaporated with the water in the flash/dryer unit. This material can be condensed in a shell-and-tube condenser and can be routed back to the process fluid while the temperature is regulated below the methanol/water vapor dew point. In so doing, it remains as a vapor and is routed out of the system.

In alternative embodiments, the dry biodiesel product **328** leaving the flash separation unit **326** is transferred to a distillation column **329** wherein the distillation column **329** is configured such that the vapor stream generated in the distillation column **330** comprises FAME and the FAME is comprised primarily of methyl palmitate and other light FAME molecules, i.e. those FAME molecules with a vapor pressure higher than that of palmitic acid. In alternative embodiments, the vapor stream comprising light FAME molecules (FAME molecules with 16 or fewer carbons) **330** can be further processed to generate product streams of individual FAMEs, e.g. a product stream of purified methyl palmitate.

In alternative embodiments, the bottoms stream of the distillation column **331** comprises those products in the first lipid stream with vapor pressures lower than methyl palmitate including, for example, "heavy" FAMEs (those FAMEs with more than 16 carbons), unreacted FFAs, unreacted esters e.g. glycerides, and any unsaponifiable material. In alternative embodiments, the bottoms stream **331** can be further process to separate discreet, high-value product streams e.g. a substantially pure vitamin E product, a substantially pure sterol product, a substantially pure squalene product, or other product streams.

In alternative embodiments, either before or after discreet product streams have been isolated from the bottoms stream **331** comprising the heavy unreacted, saturated FFAs, the remaining products are subjected to a second "polishing" i.e. esterification/ transesterification reaction **332,** either acid-catalyzed (e.g. using a strong cation exchange resin packed in a pipe) or non-catalytic, to convert the majority, i.e. 95% or more, of the unreacted FFAs and unreacted esters, e.g. glycerides, from the first esterification/transesterification reaction, to FAME or other equivalent alkyl ester if an alcohol other than methanol is used in the reaction. By subjecting the bottoms stream generated from the distillation described above to a second esterification reaction, the problem of heavy FAME molecules not being easily separated from unsaturated FFAs with similar vapor pressures (primarily C18 FAMEs and palmitic acid, if PFAD is the feedstock) is overcome. In alternative embodiments, the second esterification/ transesterification **332** reaction serves to convert approximately 95% of the unreacted FFAs and esters from the first esterification/transesterification reaction **314** into a "crude" FAME product **333** (or equivalent alkyl esters if an alcohol other than methanol is used), which, after undergoing a second distillation **334** as described above, generates a substantially purified FAME product **335** that can be blended with the purified FAME product from the first reaction **330.** In this way, the overall biodiesel yields of the process justify the use of feedstocks comprising high FFA content, wherein a large portion of the fatty acid profile, e.g., about 40% to 55% of the fatty acids in the feedstock, are saturated fatty acids.

In alternative embodiments, the second esterification/transesterification reaction proceeds identically to the first esterification/transesterification reaction but wherein the feedstock for the reaction is the "bottoms" product generated from the distillation of the lipid stream generated in the first esterification/transesterification reaction. In alternative embodiments, following the second esterification/ transesterification reaction, the generated reaction product is subjected to the same processing steps described above following the first esterification/transesterification reaction to generate a substantially purified FAME product that meets or exceeding the standards established for ASTM B100-grade biodiesel.

The invention will be further described with reference to the following examples; however, it is to be understood that the exemplary embodiments provided herein are or the invention are not limited to such examples.

### EXAMPLES

### Corn Stillage Oil Reaction (not part of the present invention)

The lipid feedstock is first combined with deionized water. The molar ratio of water to the oil is 40: 1. After combination, an emulsion is formed via mechanical sheer. The solution is then pumped into a Plug Flow Reactor via a positive displacement pump up to a pressure of 345 bar (2000 psig) (created hydraulically by compacting the fluid against a Back Pressure Regulator Valve). The contents are then heated to a temperature of 285 deg C for 20 minutes. After reaction, the incoming process material that has just left the discharge of the highpressure pump cools the already reacted solution to 95 deg C (still under ~ 345 bar (2000 psig))--this is done for heat recovery purposes. The solution then passes through a back-pressure regulator device that decreases the pressure to near one atmosphere.

After the pressure has been relieved, the liquid FFA/oil/water solution is sent to a decanter where the FFA/oil phase is separated from the heavier water/glycerol phase. The FFA/oil mixture will then be heated to a temperature of 180 deg C, and subjected to a vacuum of 0.073 bar (55 Torr) to dry any residual water off of the material. The water/glycerol phase will be sent to a packed stripping distillation column with 6 stages at atmospheric pressure producing a deionized water distillate stream which is recycled to the hydrolysis reaction to be combined with fresh lipid feedstock. The bottoms stream of the water stripper will be a splitter crude glycerol product that can be upgraded to USP with another additional distillation column.

The FFA/oil mixture is then blended with dry methanol. The mixture is emulsified via mechanical sheer and pumped into a Plug Flow Reactor via another positive displacement pump up to pressure of 345 bar (2000 psig) (created hydraulically by compacting the fluid against a Back Pressure Regulator Valve). The contents are then heated to a temperature of 285 deg C for 30 minutes. After reaction, there is another heat recovery section which cools the post-reaction fluid from the reaction temperature of 285 deg C to 215 deg C, a large amount of heat is left on the stream to ensure near complete evaporation of the unreacted methanol solvent. After the heat recovery section, the Back Pressure Regulator Valve reduces the system pressure to one atmosphere. The alcohol/water/co-solvent vapors generated during the pressure decrease are routed to an alcohol distillation column with 30 theoretical stages at atmosphere where a 99.5% or greater purity methanol stream is separated and recycled back to the process. The bottoms of the methanol column are low enough in methanol concentration (less than 200 PPM) so that normal discharge is acceptable for disposal.

After the methanol flash, the liquid Fatty Acid Methyl Ester (FAME) solution is further cooled to a temperature of 95 deg C via a shell and tube heat exchanger, this is done so that water may be added to the stream. Deionized water is then added to the process fluid at an approximate mass ratio of 1 g water: 1 g glycerol. The soft water and FAME is mixed slightly via an inline static mixer and sent to a decanter where the FAME and aqueous phases are allowed to separate. The aqueous phase is sent to a water/methanol stripper, where all of the residual alcohol is separated from the water/glycerol phase. The stripped water/methanol vapor is left uncondensed and sent back to the methanol distillation column for purification. The water/glycerol bottoms from the stripping column is combined with the feed of the distillation column that produces a splitter crude glycerol after the hydrolysis step. The FAME phase from the decanter is then heated to 180 deg C, and allowed to flash at an absolute pressure of 0.073 bar (55 Torr), this is to remove any trace amounts of solvent. The vapor removed with this evaporator is passed through a partial shell and tube condenser to capture and return any FAME that flashed along with the solvent, this material is recycled back to the feed of the evaporator. The uncondensed vapor leaving the partial shell and tube condenser has such a low amount of FAME that is condensed directly into cooling tower water via a liquid ring vacuum pump. The bottoms of the evaporator are then sent to a distillation column with 30 theoretical stages and a vacuum of 0.013 bar (10 torr). The distillate stream of that distillation column will then be collected for transport or sold, since it has a reached a purity of B100 Biodiesel.

The bottom stream from the ester distillation column will be composed of residual FFA, monoglycerides, and any unsaponifiable matter present in the original lipid feedstock. The temperature of this stream will be approximately 240 deg C. The stream is allowed to flash at 0.001 bar (1 torr) in a shortpath evaporator. This flash unit will allow any residual saponifiable material to evaporate from the unsaponifiable material. The saponified material (mainly Free Fatty Acids and Monoglycerides) will be sent through a heat exchanger and cooled. The cooled fluid is then combined with the FFA/oil mixture that is blended with methanol in the beginning of the second reaction step.-this allows the system to achieve higher yields.

### Example 12: Palm Fatty Acid Distillate Reaction

In this exemplary embodiment, the first step of this process is a non-catalytic alcohol transesterification/ esterification reaction. The reason a pretreatment hydrolysis step is not needed (compared to the Corn Stillage Oil, CSO) is due to the relatively high Free Fatty Acid (FFA) content of the Palm Fatty Acid Distillate (PFAD) compared to the CSO. The FFA material inside the lipid feedstocks is a catalyst to the transesterification reaction, however it is also a reagent in the esterification reaction. The PFAD is blended with methanol at a molar ratio of 40: 1. After combination, an emulsion is formed via mechanical sheer, which is applied via an inline rotor/stator mixer. The solution is then pumped into a Plug Flow Reactor via a positive displacement pump up to a pressure 345 bar (2000 psig) (measured at the discharge of the pump). The contents are then heated to a temperature of 285 deg C for approximately 35 minutes. After reaction, a countercurrent concentric heat exchanger is used as an economizer to recover process heat. The post-reacted fluid is cooled from 285 deg C to approximately 215 deg C. A large amount of heat is left on the stream in order to be used by the methanol for evaporation in a downstream flash unit. The 215 deg C process fluid is then allowed to flow through a Back Pressure Regulator (BPR) which reduces the pressure to one atmosphere. The methanol/water vapors generated during the pressure decrease are then routed to a distillation column with 30 theoretical stages at atmospheric pressure where a 99.5% or greater purity methanol stream is separated and recycled back to the process. The bottoms of the methanol column are low enough in methanol concentration (less than 200 PPM) so that normal discharge is acceptable for disposal.

The bottoms of the methanol flash are then further cooled via a shell and tube heat exchanger to approximately 95 deg C so that water may be used to wash the entrained glycerin out of the Fatty Acid Methyl Ester (FAME) Deionized water is then added to the process fluid at a mass ratio of 1 g water: 1 g glycerol. The soft water and FAME solution is mixed slightly via an inline static mixer and sent to a decanter where the FAME and aqueous phases are allowed to separate. The aqueous phase is sent to a methanol/water stripper, where the residual solvent is evaporated off the water/glycerol and sent back to the methanol distillation column for purification. The water/glycerin material leaving the bottom of the stripper is classified as splitter crude glycerin, and can be further upgraded to USP glycerin with the use of another distillation column.

The FAME phase from the decanter is then heated via a heat exchanger to 180 deg C, and allowed to flash at 0.073 bar (55 Torr) this is to remove any trace amounts of solvent. The vapor removed with this evaporator is passed through a partial shell and tube condenser to capture and return any FAME that flashed along with the solvent, this material is recycled back to the feed of the evaporator. The uncondensed vapor leaving the partial shell and tube condenser has such a low amount of FAME that is condensed directly into cooling tower water via a liquid ring vacuum pump. The bottoms of the evaporator (composed primarily of alkyl esters, 3-5% FFA, and unsaponifiable matter) is sent to a specialty packed distillation column with 5 theoretical stages at 0.013 bar (10 torr) producing a distillate stream comprised of 99% purity, methyl palmitate. The bottoms stream from the column composed of alkyl esters, FFAs, and unsaponifiable matter that have a vapor pressure lower than methyl palmitate continue to the second non-catalytic esterification/transesterification reactor. This step is identical to the reaction configuration in the first step of the process, except for substantially reduced volume due to the much lower residence time required for equilibrium to be reached. The stream is blended with fresh methanol. The mixture is pumped via high pressure diaphragm pump into a separate plug flow reactor and allowed to react at a pressure of 345 bar (2000 psig) and 285 deg C for 10 minutes. Following the reaction, the alcohol/water is evaporated off and sent on to the alcohol distillation column to purify and recycle the alcohol. The alkyl ester oil (now containing <1% FFA) is sent to a packed distillation column 15 theoretical stages and a vacuum range of 0013 bar (10 torr) producing a ASTM B100 biodiesel distillate and a bottoms stream that has the option to be sent on to further separation methods where nonsaponifiables can be isolate/purified.

The reason only methyl palmitate is stripped off is due to the high palmitic acid content in PFAD (compared to CSO) as well as the similarity of the vapor pressure of palmitic acid compared to all constituents of the FAME product. By removing the methyl palmitate and reacting the material again (for a short time) the equilibrium amount of palmitic acid can be dramatically reduced, making for both higher yields as well as a less complex biodiesel distillation.

### Example 2: Crude Palm Oil Reaction

In this exemplary embodiment, the Crude Palm Oil (CPO) is first combined with deionized water. The molar ratio of water to the oil is 40: 1. After combination, an emulsion is formed via mechanical sheer. The solution is then pumped into a Plug Flow Reactor via a positive displacement pump up to a pressure of 345 bar (2000 psig) (created hydraulically by compacting the fluid against a Back Pressure Regulator Valve). The contents are then heated to a temperature of 285 deg C for 20 minutes. After reaction, the incoming process material that has just left the discharge of the highpressure pump cools the already reacted solution to 95 deg C (still under ~ 345 bar (2000 psig))--this is done for heat recovery purposes. The solution then passes through a back-pressure regulator device that decreases the pressure to near one atmosphere.

After the pressure has been relieved, the liquid FFA/oil/water solution is sent to a decanter where the FFA/oil phase is separated from the heavier water/glycerol phase. The FFA/oil mixture will then be heated to a temperature of 180 deg C, and subjected to a vacuum of 0.073 bar (55 Torr) to dry any residual water off of the material. The water/glycerol phase will be sent to a packed stripping distillation column with 6 stages at atmospheric pressure producing a deionized water distillate stream which is recycled to the hydrolysis reaction to be combined with fresh lipid feedstock. The bottoms stream of the water stripper will be a splitter crude glycerol product that can be upgraded to USP with another additional distillation column.

The FFA/oil mixture is then blended with dry methanol. The mixture is emulsified via mechanical sheer and pumped into a Plug Flow Reactor via another positive displacement pump up to pressure of 345 bar (2000 psig) (created hydraulically by compacting the fluid against a Back Pressure Regulator Valve). The contents are then heated to a temperature of 285 deg C for 30 minutes. After reaction, there is another heat recovery section which cools the post-reaction fluid from the reaction temperature of 285 deg C to 215 deg C, a large amount of heat is left on the stream to ensure near complete evaporation of the unreacted methanol solvent. After the heat recovery section, the Back Pressure Regulator Valve reduces the system pressure to one atmosphere. The alcohol/water/co-solvent vapors generated during the pressure decrease are routed to an alcohol distillation column with 30 theoretical stages at atmosphere where a 99.5% or greater purity methanol stream is separated and recycled back to the process. The bottoms of the methanol column are low enough in methanol concentration (less than 200 PPM) so that normal discharge is acceptable for disposal.

The bottoms of the methanol flash are then further cooled via a shell and tube heat exchanger to approximately 95 deg C so that water may be used to wash the entrained glycerin out of the Fatty Acid Methyl Ester (FAME). Deionized water is then added to the process fluid at a mass ratio of 1 g water: 1 g glycerol. The soft water and FAME solution is mixed slightly via an inline static mixer and sent to a decanter where the FAME and aqueous phases are allowed to separate. The aqueous phase is sent to a methanol/water stripper, where the residual solvent is evaporated off the water/glycerol and sent back to the methanol distillation column for purification. The water/glycerin material leaving the bottom of the stripper is classified as splitter crude glycerin, and can be further upgraded to USP glycerin with the use of another distillation column.

The FAME phase from the decanter is then heated via a heat exchanger to 180 deg C, and allowed to flash at 0.073 bar (55 Torr) this is to remove any trace amounts of solvent. The vapor removed with this evaporator is passed through a partial shell and tube condenser to capture and return any FAME that flashed along with the solvent, this material is recycled back to the feed of the evaporator. The uncondensed vapor leaving the partial shell and tube condenser has such a low amount of FAME that is condensed directly into cooling tower water via a liquid ring vacuum pump. The bottoms of the evaporator (composed primarily of alkyl esters, 3-5% FFA, and unsaponifiable matter) is sent to a specialty packed distillation column with 5 theoretical stages at 0.013 bar (10 torr) producing a distillate stream comprised of 99% purity, methyl palmitate.

The bottoms stream from the column composed of alkyl esters, FFAs, and unsaponifiable matter that have a vapor pressure lower than methyl palmitate continue to the second non-catalytic esterification/transesterification reactor. This step is identical to the reaction configuration in the first step of the process, except for substantially reduced volume due to the much lower residence time required for equilibrium to be reached. The stream is blended with fresh methanol. The mixture is pumped via high pressure diaphragm pump into a separate plug flow reactor and allowed to react at a pressure of 345 bar (2000 psig) and 285 deg C for 10 minutes. Following the reaction, the alcohol/water is evaporated off and sent on to the alcohol distillation column to purify and recycle the alcohol. The alkyl ester oil (now containing <1% FFA) is sent to a packed distillation column 15 theoretical stages and a vacuum range of 0.013 bar (10 torr) producing a ASTM B100 biodiesel distillate and a bottoms stream that has the option to be sent on to further separation methods where nonsaponifiables can be isolate/purified.

The reason only methyl palmitate is stripped off is due to the high palmitic acid content in CPO (compared to CSO) as well as the similarity of the vapor pressure of palmitic acid compared to all constituents of the FAME product. By removing the methyl palmitate and reacting the material again (for a short time) the equilibrium amount of palmitic acid can be dramatically reduced, making for both higher yields as well as a less complex biodiesel distillation.

### Mass balance of corn stillage oil (CSO) reaction (not part of the present invention)

The following tables show the mass-balance of an exemplary embodiment of the 2-stage process. In the present example, corn stillage oil (CSO) is reacted in a first hydrolysis stage to generate an FFA-comprising lipid phase. The FFA-comprising lipid phase is then reacted in a second esterification stage wherein the FFA-comprising lipid phase from the first reaction was reacted with methanol at a temperature and pressure above the supercritical temperature and pressure of the alcohol. Mass-balance of the process is shown below (units for al numbers are Metric Tons):

| | Feed to reactor | Post hydrolysis reactor | Feed to decanter | Non-polar (aqueous) Phase of Decanter | Polar (lipid) phase of decanter | Distillate of water stripper | Bottoms of water stripper |
|---|---|---|---|---|---|---|---|
| Methanol | 82 | 77 | 77 | | | | |
| Water | 88 | 0 | | 0.5 | 76.5 | 75.2 | 1.3 |
| Triglycerides | | 2 | 2 | | | | |
| Diglycerides | | 5 | 5 | 2 | | | |
| Monoglycerides | | | | 5 | | | |
| Fatty Acid Methyl Ester | 10 | 90 | 90 | | | | |
| Free Fatty Acid | | 6 | 6 | 90 | | | |
| Glycerol | 2 | 2 | 2 | | 6 | | 6 |
| Unsaponifiables | 182 | 182 | 182 | 2 | | | |
| Total | 82 | 77 | 77 | 99.5 | 82.5 | 75.2 | 7.3 |

| | Feed to residual water flash | Vapor of Water flash | Bottoms of water flash | Feed to methanol reactor | Post-reaction methanol reactor |
|---|---|---|---|---|---|
| Methanol | | | | 66 | 56 |
| Water | 0.5 | 0.49 | 0.01 | 1 | 2.5 |
| Triglycerides | | | | | |
| Diglycerides | 2 | | 2 | 2 | 0.5 |
| Monoglycerides | 5 | | 5 | 5 | 2 |
| Fatty Acid Methyl Ester | | | | | 100.5 |
| Free Fatty Acid | 90 | | 90 | 90 | 2 |
| Glycerol | | | | | 0.5 |
| Unsaponifiables | 2 | | 2 | 2 | 2 |
| Total | 99.5 | 0.49 | 99.01 | 166 | 166 |

| | Vapor of methanol flash | Bottoms of methanol flash | Feed to decanter | Non-polar Phase of Decanter | Polar phase of decanter |
|---|---|---|---|---|---|
| Methanol | 54 | 2 | 2 | 0.5 | 1.5 |
| Water | 2.4 | 0.1 | 0.1 | 0.05 | 0.05 |
| Triglycerides | | | | | |
| Diglycerides | | 0.5 | 0.5 | 0.5 | |
| Monoglycerides | | 2 | 2 | 2 | |
| Fatty Acid Methyl Ester | | 100.5 | 100.5 | 100.5 | |
| Free Fatty Acid | | 2 | 2 | 2 | |
| Glycerol | | 0.5 | 0.5 | | 0.5 |
| Unsaponifiables | | 2 | 2 | 2 | |
| Total | 56.4 | 109.6 | 109.6 | 107.55 | 2.05 |

| | Vapor of residual solvent flash | Bottoms of residual solvent flash | Feed to fame column | Vapor of fame column |
|---|---|---|---|---|
| Methanol | 0.49 | 0.01 | 0.01 | 0.01 |
| Water | 0.04 | 0.01 | 0.01 | 0.01 |
| Triglycerides | | | | |
| Diglycerides | | 0.5 | 0.5 | |
| Monoglycerides | | 2 | 2 | |
| Fatty Acid Methyl Ester | | 100.5 | 100.5 | 100.4 |
| Free Fatty Acid | | 2 | 2 | 0.1 |
| Glycerol | | | | |
| Unsaponifiables | | 2 | 2 | |
| Total | 0.53 | 107.02 | 107.02 | 100.52 |

| | Feed to shortpath unit | Vapor (recycle to shortpath unit) | Bottoms of shortpath unit |
|---|---|---|---|
| Methanol | | | |
| Water | | | |
| Triglycerides | | | |
| Diglycerides | 0.5 | | 0.5 |
| Monoglycerides | 2 | 1.8 | 0.2 |
| Fatty Acid Methyl Ester | 0.1 | 0.1 | |
| Free Fatty Acid | 1.9 | 1.7 | 0.2 |
| Glycerol | | | |
| Unsaponifiables | 2 | | 2 |
| Total | 6.5 | 3.6 | 2.9 |

While the forgoing written description enables one of ordinary skill to make and use alternative embodiments including a best mode thereof, those of ordinary skill will understand and appreciate the existence of variations, combinations, and equivalents of the specific embodiments, methods, and examples herein. The invention should therefore not be limited by the above described exemplary embodiments, methods and examples, but by all embodiments and methods within the scope of the invention.

## Claims

1. A method or an industrial process for producing a biodiesel or a fat-based diesel fuel from a feedstock comprising a palm oil fatty acid distillate (PFAD) or a feedstock comprising a palm oil, dende oil, an oil from a plant of the genus *Elaeis* or *Attalea,* the method comprising:
(a) providing a palm oil fatty acid distillate (PFAD) or a feedstock comprising a palm oil, dende oil, an oil from a plant of the genus Elaeis or Attalea ;
(b) providing an alcohol;
(c) subjecting the PFAD or feedstock of (a) to an esterification/ transesterification reaction with the alcohol under conditions comprising at or above the critical temperature and pressure of the alcohol in the absence of any catalyst, wherein free fatty acids (FFAs) in the PFAD or feedstock undergo an esterification reaction with the alcohol to generate a product comprising fatty acid alkyl esters, and the glycerides undergo a transesterification reaction with the alcohol to generate a product comprising fatty acid alkyl esters; and
(d) separating the product generated in the esterification/transesterification reaction of step (c) into a "light" fraction comprising the lighter alkyl esters, optionally alkyl esters with 16 or fewer carbons, and a "heavy" fraction comprising heavy alkyl esters, optionally alkyl esters with more than 16 carbons, and any unreacted FFAs.

2. The method of claim 1, wherein in step b) the alcohol comprises a methanol.

3. The method or industrial process of claim 1 wherein the esterification/transesterification reaction product is distilled, optionally in a conventional distillation column or equivalent, to separate the lighter fatty acid alkyl esters from the other components of the reaction product.

4. The method or industrial process of claim 1 wherein if PFAD is the feedstock, the majority of the fatty acid alkyl esters comprise alkyl esters of palmitic acid, optionally methyl palmitate if methanol is the alcohol used in the reaction, and the majority of the fatty acids present in the feedstock with 16 or fewer carbons comprise palmitic acid.

5. The method or industrial process of claim 1 wherein a bottoms fraction in the distillation column comprises heavy fatty acid alkyl esters, and optionally the bottoms fraction comprises alkyl esters with more than 16 carbons, unreacted FFAs, any unreacted esters e.g. mono- di- and triglycerides, phospholipids, and any other unsaponifiable material in the feedstock, optionally sterols, vitamin E compounds, and optionally the vitamin E compounds comprise tocopherols and/or tocotrienols or squalene.

6. The method or industrial process of claim 1, further comprising subjecting the "heavy" fraction comprising heavy alkyl esters, or the bottoms of the distillation column, to a second esterification/transesterification reaction with a supercritical alcohol, or at or above the critical temperature and pressure of the alcohol in the absence of any catalyst,
wherein approximately 95% of the unreacted FFAs and esters from the first esterification/transesterification reaction are converted to fatty acid alkyl esters,
and optionally the product mixture generated in the second esterification/ transesterification reaction generates a product mixture comprising less than about 1% FFA.

7. The method or industrial process of anyone of claims 1-6, further comprising processing the second product mixture to separate the fatty acid alkyl esters from the remaining components of the product mixture using, optionally distilling or separating to generate an alkyl ester product that is suitable for use as an ASTM B100 biodiesel.

8. The method or industrial process of anyone of claims 1-7, wherein the alkyl ester biodiesel product separated in the second distillation or other separation technique are mixed or combined with the alkyl esters separated from the first reaction product to increase the overall biodiesel yield of the process.

9. The method or industrial process of anyone of claims 1-8, further comprising subjecting the "heavy" fraction comprising heavy alkyl esters, or the bottoms of the distillation column, to an acid-catalyzed alcohol esterification reaction (instead of a second esterification/ transesterification reaction with a supercritical alcohol) comprising a strong acid cation exchange resin, wherein optionally the reaction is an alcohol esterification reaction in the presence of a strong acid cation resin or equivalent, the resin acting as an acid catalyst of the reaction.

10. The method or industrial process of claim 8, further comprising:
(i)
(a) mixing the bottoms (comprising the unreacted FFAs, any unreacted esters, optionally mono- di- and triglycerides, phospholipids, and any other unsaponifiable material in the feedstock, optionally sterols, vitamin E compounds such as tocopherols and/or tocotrienols, squalene, or other compounds, with an alcohol, optionally methanol, to form an alcohol/bottoms mixture; and
(b) heating the alcohol/bottoms mixture, optionally using a heat exchanger, optionally, a heat exchanger operationally connected to another portion of the process, to between about 80 and 100 °C;
(ii) passing or pumping the alcohol/bottoms mixture through a pipe or other suitable container or vessel comprising a cation resin (optionally a packed cation resin) or equivalent until substantially all of the saponifiable material in the mixture is converted to fatty acid alkyl esters; or
(iii) flashing off unreacted alcohol, and optionally recovering and recycling the alcohol.

## Patentansprüche

1. Verfahren oder industrieller Prozess zum Herstellen eines Biodiesels oder eines Dieselkraftstoffs auf Fettbasis aus einem Ausgangsmaterial, das ein Palmöl-Fettsäuredestillat (PFAD) oder ein Ausgangsmaterial mit einem Palmöl, Dendeöl, einem Öl aus einer Pflanze der Gattung Elaeis oder Attalea enthält, wobei das Verfahren folgende Schritte umfasst:
(a) Bereitstellen eines Palmöl-Fettsäuredestillats (PFAD) oder eines Ausgangsmaterials, das Palmöl, Dende-Öl oder ein Öl aus einer Pflanze der Gattung Elaeis oder Attalea enthält;
(b) Bereitstellen eines Alkohols;
(c) Unterziehen des PFAD oder Ausgangsmaterials von (a) einer Veresterungs-/Umesterungsreaktion mit dem Alkohol unter Bedingungen, die bei oder über der kritischen Temperatur und dem kritischen Druck des Alkohols liegen, in Abwesenheit irgendeines Katalysators, wobei freie Fettsäuren (FFAs) in dem PFAD oder Ausgangsmaterial eine Veresterungsreaktion mit dem Alkohol eingehen, um ein Produkt zu erzeugen, das Fettsäurealkylester enthält, und die Glyceride einer Umesterungsreaktion mit dem Alkohol eingehen, um ein Produkt zu erzeugen, das Fettsäurealkylester enthält; und
(d) Trennen des in der Veresterungs-/Umesterungsreaktion von Schritt (c) erzeugten Produkts in eine "leichte" Fraktion, welche die leichteren Alkylester, optional Alkylester mit 16 oder weniger Kohlenstoffen, enthält, und eine "schwere" Fraktion, die schwere Alkylester, optional Alkylester mit mehr als 16 Kohlenstoffen, und etwaige nicht umgesetzte FFAs enthält.

2. Verfahren nach Anspruch 1, wobei der Alkohol in Schritt b) Methanol umfasst.

3. Verfahren oder industrieller Prozess nach Anspruch 1, wobei das Veresterungs-/Umesterungsreaktionsprodukt optional in einer herkömmlichen Destillationskolonne oder einem Äquivalent destilliert wird, um die leichteren Fettsäurealkylester von den anderen Komponenten des Reaktionsprodukts zu trennen.

4. Verfahren oder industrieller Prozess nach Anspruch 1, wobei, wenn PFAD das Ausgangsmaterial ist, die Mehrheit der Fettsäurealkylester Alkylester der Palmitinsäure, optional Methylpalmitat, wenn Methanol der in der Reaktion verwendete Alkohol ist, enthält, und die Mehrheit der im Ausgangsmaterial vorhandenen Fettsäuren mit 16 oder weniger Kohlenstoffatomen Palmitinsäure enthält.

5. Verfahren oder industrieller Prozess nach Anspruch 1, wobei eine Bodenfraktion in der Destillationssäule schwere Fettsäurealkylester enthält, und die Bodenfraktion optional Alkylester mit mehr als 16 Kohlenstoffatomen, nicht umgesetzte FFAs, etwaige nicht umgesetzte Ester, z.B. Mono-, Di- und Triglyceride, Phospholipide und jedes andere unverseifbare Material im Ausgangsmaterial, optional Sterole, Vitamin-E-Verbindungen enthält, und die Vitamin-E-Verbindungen optional Tocopherole und/oder Tocotrienole oder Squalen enthalten.

6. Verfahren oder industrieller Prozess nach Anspruch 1, ferner umfassend das Unterziehen der "schweren" Fraktion, die schwere Alkylester enthält, oder des Bodensatzes der Destillationssäule einer zweiten Veresterungs-/Umesterungsreaktion mit einem überkritischen Alkohol, oder bei oder über der kritischen Temperatur und dem kritischen Druck des Alkohols in Abwesenheit irgendeines Katalysators,
wobei etwa 95 % der nicht umgesetzten FFAs und Ester aus der ersten Veresterungs-/Umesterungsreaktion in Fettsäurealkylester umgewandelt werden, und optional das in der zweiten Veresterungs-/Umesterungsreaktion erzeugte Produktgemisch ein Produktgemisch erzeugt, das weniger als etwa 1 % FFA enthält.

7. Verfahren oder industrieller Prozess nach einem der Ansprüche 1 bis 6, das ferner das Verarbeiten des zweiten Produktgemischs umfasst, um die Fettsäurealkylester von den übrigen Bestandteilen des Produktgemischs, optional unter Verwendung eines Destillierens oder Trennens, zu trennen, um ein Alkylesterprodukt zu erzeugen, das zur Verwendung als ASTM B100-Biodiesel geeignet ist.

8. Verfahren oder industrieller Prozess nach einem der Ansprüche 1 bis 7, wobei das in der zweiten Destillation oder einem weiteren Trennverfahren abgetrennte Alkylester-Biodieselprodukt mit den aus dem ersten Reaktionsprodukt abgetrennten Alkylestern gemischt oder kombiniert wird, um die Biodiesel-Gesamtausbeute des Prozesses zu erhöhen.

9. Verfahren oder industrieller Prozess nach einem der Ansprüche 1 bis 8, das zudem das Unterziehen der "schweren" Fraktion, die schwere Alkylester enthält, oder des Bodensatzes der Destillationssäure einer säurekatalysierten Alkoholveresterungsreaktion (anstelle einer zweiten Veresterungs-/Umesterungsreaktion) mit einem überkritischen Alkohol) umfasst, die ein stark saures Kationenaustauscherharz enthält, wobei die Reaktion optional eine Alkoholveresterungsreaktion in Gegenwart eines stark sauren Kationenharzes oder eines Äquivalents ist, wobei das Harz als Säurekatalysator der Reaktion wirkt.

10. Verfahren oder industrieller Prozess nach Anspruch 8, ferner umfassend:
(i)
(a) Mischen des Bodensatzes (der die nicht umgesetzten FFAs, etwaige nicht umgesetzten Ester, optional Mono-, Di- und Triglyceride, Phospholipide und jedes andere unverseifbare Material im Ausgangsmaterial, optional Sterole, Vitamin-E-Verbindungen wie z. B. Tocopherole und/oder Tocotrienole, Squalen oder andere Verbindungen enthält) mit einem Alkohol, optional Methanol, um ein Alkohol-/Bodensatzgemisch zu bilden; und
(b) Erhitzen des Alkohol-/Bodensatzgemischs, optional unter Verwendung eines Wärmetauschers, optional eines Wärmetauschers, der mit einem anderen Abschnitt des Prozesses wirkverbunden ist, auf etwa 80 bis 100 °C;
(ii) Leiten oder Pumpen des Alkohol-/Bodensatzgemischs durch ein Rohr oder einen anderen geeigneten Behälter oder ein Gefäß, das ein Kationenharz (optional ein gepacktes Kationenharz) oder ein Äquivalent enthält, bis das im Wesentlichen gesamte verseifbare Material im Gemisch in Fettsäurealkylester umgewandelt ist; oder
(iii) Abdunsten von nicht umgesetztem Alkohol, und optional Rückgewinnen und Recyceln des Alkohols.

## Revendications

1. Procédé ou processus industriel de production d'un biodiesel ou d'un carburant diesel à base de graisse à partir d'une matière première comprenant un distillat d'acides gras d'huile de palme (PFAD) ou une matière première comprenant une huile de palme, une huile de dendê, une huile issue d'une plante du genre *Elaeis* ou *Attalea,* le procédé comprenant :
(a) utiliser un distillat d'acides gras d'huile de palme (PFAD) ou une matière première comprenant une huile de palme, une huile de dendê, une huile issue d'une plante du genre *Elaeis* ou *Attalea ;*
(b) utiliser un alcool ;
(c) soumettre le PFAD ou la matière première de (a) à une réaction d'estérification/transestérification avec l'alcool dans des conditions comprenant une température et une pression égales ou supérieures à la température critique et à la pression critique de l'alcool en l'absence de tout catalyseur, dans lequel des acides gras libres (FFA) du PFAD ou de la matière première subissent une réaction d'estérification avec l'alcool pour générer un produit comprenant des esters alkyliques d'acides gras, et les glycérides subissent une réaction de transestérification avec l'alcool pour générer un produit comprenant des esters alkyliques d'acides gras ; et
(d) séparer le produit généré lors de la réaction d'estérification/transestérification de l'étape (c) en une fraction « légère » comprenant les esters alkyliques plus légers, éventuellement des esters alkyliques comportant au maximum 16 atomes de carbone, et une fraction « lourde » comprenant des esters alkyliques lourds, éventuellement des esters alkyliques comportant plus de 16 atomes de carbone, et de quelconques FFA n'ayant pas réagi.

2. Procédé selon la revendication 1, dans lequel, à l'étape (b), l'alcool comprend un méthanol.

3. Procédé ou processus industriel selon la revendication 1, dans lequel le produit de réaction d'estérification/transestérification est distillé, éventuellement dans une colonne de distillation classique, ou un équivalent, pour séparer les esters alkyliques d'acides gras plus légers des autres constituants du produit de réaction.

4. Procédé ou processus industriel selon la revendication 1, dans lequel, si le PFAD est la matière première, la majeure partie des esters alkyliques d'acides gras comprend des esters alkyliques d'acide palmitique, éventuellement du palmitate de méthyle si du méthanol est l'alcool utilisé lors de la réaction, et la majeure partie des acides gras présents dans la matière première comportant au maximum 16 atomes de carbone comprend de l'acide palmitique.

5. Procédé ou processus industriel selon la revendication 1, dans lequel une fraction de résidu dans la colonne de distillation comprend des esters alkyliques d'acides gras lourds, et éventuellement la fraction de résidu comprend des esters alkyliques comportant plus de 16 atomes de carbone, des FFA n'ayant pas réagi, de quelconques esters n'ayant pas réagi, par exemple des mono-, di- et triglycérides, des phospholipides, et toute autre matière non saponifiable de la matière première, éventuellement des stérols, des composés de vitamines E, et les composés de vitamines E comprennent éventuellement des tocophérols et/ou des tocotriénols ou du squalène.

6. Procédé ou processus industriel selon la revendication 1, comprenant en outre soumettre la fraction « lourde » comprenant des esters alkyliques lourds, ou le résidu de la colonne de distillation, à une seconde réaction d'estérification/transestérification avec un alcool supercritique, ou à une température et une pression supérieures ou égale à la température critique et à la pression critique de l'alcool en l'absence d'un quelconque catalyseur,
dans lequel sensiblement 95 % des FFA et des esters n'ayant pas réagi issus de la première réaction d'estérification/transestérification sont convertis en esters alkyliques d'acides gras,
et éventuellement le mélange de produits généré lors de la seconde réaction d'estérification/transestérification génère un mélange de produits comprenant moins d'environ 1 % de FFA.

7. Procédé ou processus industriel selon l'une quelconque des revendications 1 à 6, comprenant en outre traiter le second mélange de produits pour séparer les esters alkyliques d'acides gras des constituants restants du mélange de produits au moyen éventuellement d'une distillation ou d'une séparation pour générer un produit d'esters alkyliques qui est approprié pour une utilisation en tant que biodiesel B100 de l'ASTM.

8. Procédé ou processus industriel selon l'une quelconque des revendications 1 à 7, dans lequel le produit de biodiesel d'esters alkyliques séparé lors de la seconde distillation ou par une autre technique de séparation est mélangé ou combiné avec les esters alkyliques séparés du premier produit de réaction pour augmenter le rendement de biodiesel global du processus.

9. Procédé ou processus industriel selon l'une quelconque des revendications 1 à 8, comprenant en outre soumettre la fraction « lourde » comprenant des esters alkyliques lourds, ou le résidu de la colonne de distillation, à une réaction d'estérification d'alcool catalysé par acides (à la place d'une seconde réaction d'estérification/transestérification avec un alcool supercritique) comprenant une résine échangeuse de cations fortement acide, dans lequel la réaction est éventuellement une réaction d'estérification d'alcool en la présence d'une résine cationique fortement acide ou d'un équivalent, la résine agissant en tant que catalyseur acide de la réaction.

10. Procédé ou processus industriel selon la revendication 8, comprenant en outre :
(i)
(a) mélanger le résidu (comprenant les FFA n'ayant pas réagi, de quelconques esters n'ayant pas réagi, éventuellement des mono-, di- et triglycérides, des phospholipides, et toute autre matière non saponifiable de la matière première, éventuellement des stérols, des composés de vitamines E tels que des tocophérols et/ou des tocotriénols ou du squalène, ou d'autres composés, avec un alcool, éventuellement du méthanol, pour former un mélange alcool/résidu ; et
(b) chauffer le mélange alcool/résidu, éventuellement au moyen d'un échangeur de chaleur, éventuellement, un échangeur de chaleur connecté de manière fonctionnelle à une autre partie du processus, à une température comprise entre environ 80 et 100 °C ;
(ii) faire passer ou pomper le mélange alcool/résidu à travers une conduite ou un autre contenant ou récipient approprié comprenant une résine cationique (éventuellement une résine cationique compacte) ou un équivalent jusqu'à ce que sensiblement l'ensemble de la matière saponifiable du mélange soit convertie en esters alkyliques d'acides gras ;
ou
(iii) vaporiser par détente l'alcool n'ayant pas réagi, et éventuellement récupérer et recycler l'alcool.
